# EUROPEAN PATENT SPECIFICATION

(11) **EP 3 976 028 B1**
(45) Date of publication and mention of the grant of the patent: **24.06.2026**
(21) Application number: 20731006.1
(22) Date of filing: 29.05.2020
(51) Int. Cl.: A61K 31/4245, A61P 25/02

(54) **SUBSTITUTED 4-[5-(BENZOFURAN-2-YL)-1,2,4-OXADIAZOL-3-YL]BENZOIC ACID COMPOUNDS FOR USE IN THERAPY FOR NEUROPATHIC PAIN**
SUBSTITUIERTE 4-[5-(BENZOFURAN-2-YL)-1,2,4-OXADIAZOL-3-YL]BENZOESÄURE-VERBINDUNGEN ZUR VERWENDUNG IN DER THERAPIE VON NEUROPATHISCHEN SCHMERZEN
COMPOSÉS D'ACIDE 4-[5-(BENZOFURAN-2-YL)-1,2,4-OXADIAZOL-3-YL] BENZOÏQUE SUBSTITUÉ DESTINÉS À ÊTRE UTILISÉS DANS LE TRAITEMENT DE LA DOULEUR NEUROPATHIQUE

(30) Priority: 30.05.2019 GB 201907647
(43) Date of publication of application: 06.04.2022
(73) Proprietor: King's College London, London WC2R 2LS (GB)
(72) Inventor: CORCORAN, Jonathan Patrick Thomas, London Greater London SE1 1UL (GB); DE CASTRO VASCONCELOS GONCALVES, Maria Beatriz, London Greater London SE1 1UL (GB)
(74) Representative: Mewburn Ellis LLP
(86) International application number: PCT/EP2020/064969
(87) International publication number: WO 2020/239978

(56) References cited:
- WO-A1-2016/097004
- WO-A1-2017/220446
- WO-A2-2008/057930
- GUI YULONG ET AL: "Bexarotent Attenuated Chronic Constriction Injury-Induced Spinal Neuroinflammation and Neuropathic Pain by Targeting Mitogen-Activated Protein Kinase Phosphatase-1", JOURNAL OF PAIN, SAUNDERS, PHILADELPHIA, PA, US, vol. 21, no. 11, 18 January 2019 (2019-01-18), pages 1149 - 1159, XP086380927, ISSN: 1526-5900, [retrieved on 20190118], DOI: 10.1016/J.JPAIN.2019.01.007
- MARIA B. GONCALVES ET AL: "Discovery and lead optimisation of a potent, selective and orally bioavailable RAR[beta] agonist for the potential treatment of nerve injury", BIOORGANIC & MEDICINAL CHEMISTRY LETTERS, vol. 29, no. 8, 11 February 2019 (2019-02-11), AMSTERDAM, NL, pages 995 - 1000, XP055722005, ISSN: 0960-894X, DOI: 10.1016/j.bmcl.2019.02.011
- MARIA B GONCALVES ET AL: "RARb Agonist Drug (C286) Demonstrates Efficacy in a Pre-clinical Neuropathic Pain Model Restoring Multiple Pathways via DNA Repair Mechanisms RARb Agonist Drug (C286) Demonstrates Efficacy in a Pre-clinical Neuropathic Pain Model Restoring Multiple Pathways via DNA Repair Mechanisms", ISCIENCE, 17 September 2019 (2019-09-17), pages 1 - 31, XP055722061, Retrieved from the Internet <URL:https://reader.elsevier.com/reader/sd/pii/S258900421930358X?token=BEC6F0D796A3E177C549ECBCF0A295A5FD6189753CE4AF9F44D00D7901DC4D37C604E7E37F2FE0A4ED463F7E6946EA77> [retrieved on 20200812]

## Description

### TECHNICAL FIELD

The present invention pertains generally to the field of therapy. More specifically, the present invention pertains to certain substituted 4-[5-(benzofuran-2-yl)-1,2,4-oxadiazol-3-yl]benzoic acid compounds (collectively referred to herein as BOBA compounds), and pharmaceutical compositions comprising those compounds, for use in a method of treatment or prevention of neuropathic pain.

### BACKGROUND

A number of publications are cited herein in order to more fully describe and disclose the invention and the state of the art to which the invention pertains.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

This disclosure includes information that may be useful in understanding the present invention. It is not an admission that any of the information provided herein is prior art or relevant to the presently claimed invention, or that any publication specifically or implicitly referenced is prior art.

### Neuropathic Pain

Neuropathic pain is a chronic pathology secondary to a lesion or disease of the somatosensory nervous system and affects 7-10% of the general population (see, e.g., Colloca *et al.,* 2017).

In the chronic pain population, neuropathic pain is found in, for example, post-herpetic neuralgia, painful diabetic polyneuropathy, post-surgery neuropathic pain, multiple sclerosis, spinal cord injury, stroke, and cancer (see, e.g., Colloca *et al.,* 2017). The clinically most important painful generalized peripheral neuropathies include those associated with diabetes mellitus, pre-diabetes, and other metabolic dysfunctions; infectious diseases (mainly HIV infection and leprosy); chemotherapy; immune and inflammatory disorders (for example, Guillain-Barré syndrome); inherited neuropathies and channelopathies (such as inherited erythromelalgia, a disorder in which blood vessels are episodically blocked then become hyperaemic and inflamed; see, e.g., Colloca *et al.,* 2017).

There are two primary features of neuropathic pain: (1) hyperalgesia - increased pain from a stimulus that usually evokes pain; and (2) allodynia - pain due to a stimulus that usually does not provoke pain (see, e.g., Jensen *et al.,* 2014).

It appears that there are at least two distinct aspects to the development of these features: (a) peripheral sensitisation, involving changes in the threshold of peripheral nociceptors including possible spontaneous firing, and (b) central sensitisation in which there are changes in the responsiveness at the central synapses relaying nociception, especially in the dorsal horn of the spinal cord.

There is still debate about the importance of central sensitisation and whether it relies, for its maintenance, on the peripherally sensitised input (see, e.g., Meacham *et al.,* 2017). Nevertheless, the occurrence of mechanical allodynia, in which Aβ mechanoceptor fibre activation causes pain, rather than its alleviation, is clear evidence for a central change, one part of which is due to the release of brain-derived neurotrophic factor (BDNF) in the dorsal horn. This appears to cause two effects: (1) mechanoceptive input is excitatory, rather than inhibitory, on the neurones relaying nociception because of a shift in the reversal potential of *E*_{GABA}; and (2) a reduction in the efficacy of presynaptic inhibition on the nociceptive afferent fibres (see, e.g., Chen *et al.,* 2014).

In keeping with these results, a single intrathecal injection of BDNF results in pain for 48 days, but subsequently resolves (see, e.g., Constandil *et al.,* 2011), thus suggesting that there is a long-lasting, albeit not permanent, central change.

### Treatment of Neuropathic Pain

Neuropathic pain is generally managed with a tricyclic antidepressant (e.g., amitriptyline, nortriptyline, desipramine) or with certain antiepileptic drugs (e.g., selective serotonin reuptake inhibitors such as paroxetine and citalopram; venlafaxine, bupropion).

Antiseizure treatments (e.g., carbamazepine, phenytoin, gapapentin, pregabalin, lamotrigine) may also be effective treatments for neuropathic pain.

Neuropathic pain may respond to opioid analgesics, such as tramadol hydrochloride, morphine, and oxycodone hydrochloride.

Patients with localised pain who are unable to take oral medicines may benefit from topical local anaesthetic preparations, such as lidocaine and capsaicin, often in patch or gel/cream form.

Neuromodulation by spinal cord stimulation may be of benefit in some patients. Many patients with chronic neuropathic pain require multidisciplinary management, including physiotherapy and psychological support.

### Novel Therapy for Neuropathic Pain (NP)

Previously, the Applicant demonstrated that various bicycloheteroaryl-heteroaryl-benzoic acid compounds act as retinoic acid receptor beta (RARβ) agonists, and cause axonal outgrowth in models of nerve injury. See, e.g., Borthwick *et al.,* 2016.

In order for neurons to communicate with each other, they need to form synapses. However, there is no evidence from other work using other axonal outgrowth molecules or interventions that the *correct* synapse connections will be formed. Therefore, treatment with compounds that cause axonal outgrowth (such as those described in Borthwick *et al.,* 2016) is expected to result in synapses that have been formed in a haphazard manner and have misconnections. It is these misconnections that cause pain. The pain may be due to inflammatory cells in the brain, such as microglia and astrocytes regulating synaptic structure and function.

The Applicant has now demonstrated that, surprisingly and unexpectedly, certain compounds that cause axonal outgrowth (described in Borthwick *et al.,* 2016) can be used to treat and prevent neuropathic pain, and may do so by downregulating inflammatory pathways.

As described herein, BOBA-001 prevents neuropathic pain in the rat; it regulates thermal and mechanical threshold and down-regulates key inflammatory and pain mediators.

Accordingly, BOBA-001, as well as the other BOBA compounds described herein, are likely to be useful for the treatment and prevention of neuropathic pain. This is surprising and unexpected because BOBA-001, as well as the other BOBA compounds described herein, also cause axonal outgrowth.

Goncalves *et al.,* 2019a, describes the discovery and lead optimisation of an RARβ agonist with the potential for treatment of nerve injury. Compound 10 therein corresponds to the compound BOBA-001 of the present application. However, nowhere therein there any mention of pain, let alone neuropathic pain.

Goncalves *et al.,* 2019b, describes studies using a particular RARβ agonist (referred to therein as C286, and corresponding to the compound BOBA-001 of the present application) in a neuropathic pain model in rats.

### Known Compounds

Borthwick *et al.,* 2016 describes certain bicycloheteroaryl-heteroaryl-benzoic acid (BHBA) compounds, their activity in (selectively) activating RARβ2 (e.g., RARβ2), and their use in the treatment of diseases and conditions that are mediated by RARβ (e.g., RARβ2), including, e.g., neurological injuries such as spinal cord injuries. Among the examples provided therein are certain substituted 4-[5-(benzofuran-2-yl)-1,2,4-oxadiazol-3-yl]benzoic acid compounds referred to as "BHBA-001" through "BHBA-020".

Borthwick *et al.,* 2017, describes the preparation and characterisation of certain crystalline forms of 4-(5-(4,7-dimethylbenzofuran-2-yl)-1,2,4-oxadiazol-3-yl)benzoic acid, referred to therein as "BHBA-001".

Chiang *et al.,* 2008, alleges that neuropathic pain may be treated with retinoic acid receptor (RAR) agonists (without specifying which subtype, e.g., RARα, RARβ, *etc.*)*.* The document merely refers to two classes of compounds (one based on tazarotene and one based on R-667) which were undergoing clinical trial studies (for the treatment of psoriasis and emphysema, respectively). The document alleges that these compounds may also be used for the treatment of pain, including neuropathic pain. The document provides no supporting data; instead, the document refers to studies that had not yet been done (see, e.g., page 14, lines 11-12 therein; "Activity of R-667 and Tazarotene will be demonstrated in the SNL model, with the following protocol").

### SUMMARY OF THE INVENTION

The present invention relates to certain substituted 4-[5-(benzofuran-2-yl)-1,2,4-oxadiazol-3-yl]benzoic acid compounds (collectively referred to herein as BOBA compounds), as described herein, and pharmaceutical compositions comprising those compounds, which are useful for the treatment or prevention of neuropathic pain, as described herein.

One aspect of the invention pertains to a BOBA compound, as described herein, or a pharmaceutical composition comprising a BOBA compound, as described herein, for use in a method of treatment or prevention of neuropathic pain.

Also described herein, but not part of the claimed invention, is use of a BOBA compound, as described herein, in the manufacture of a medicament for treatment or prevention of neuropathic pain.

Also described herein, but not part of the claimed invention, is a method of treatment or prevention of neuropathic pain, comprising administering to a patient in need of treatment a therapeutically effective amount of a BOBA compound, as described herein, or a pharmaceutical composition comprising a BOBA compound, as described herein.

Also described herein, but not part of the claimed invention, is use of a BOBA compound, as described herein, or a pharmaceutical composition comprising a BOBA compound, as described herein, in the treatment or prevention of neuropathic pain.

Also described herein, but not part of the claimed invention, is a kit comprising (a) a BOBA compound, as described herein, or a composition comprising a BOBA compound, as described herein, *e.g*., preferably provided in a suitable container and/or with suitable packaging; and (b) instructions for use, *e.g*., written instructions on how to administer the compound or composition, for the treatment or prevention of neuropathic pain.

In one embodiment, the BOBA compound or a pharmaceutical composition comprising the BOBA compound is administered orally, for example, by oral administration, orally administering, an oral medicament, *etc.*

As will be appreciated by one of skill in the art, features and preferred embodiments of one aspect of the invention will also pertain to other aspects of the invention.

### BRIEF DESCRIPTION OF THE DRAWINGS

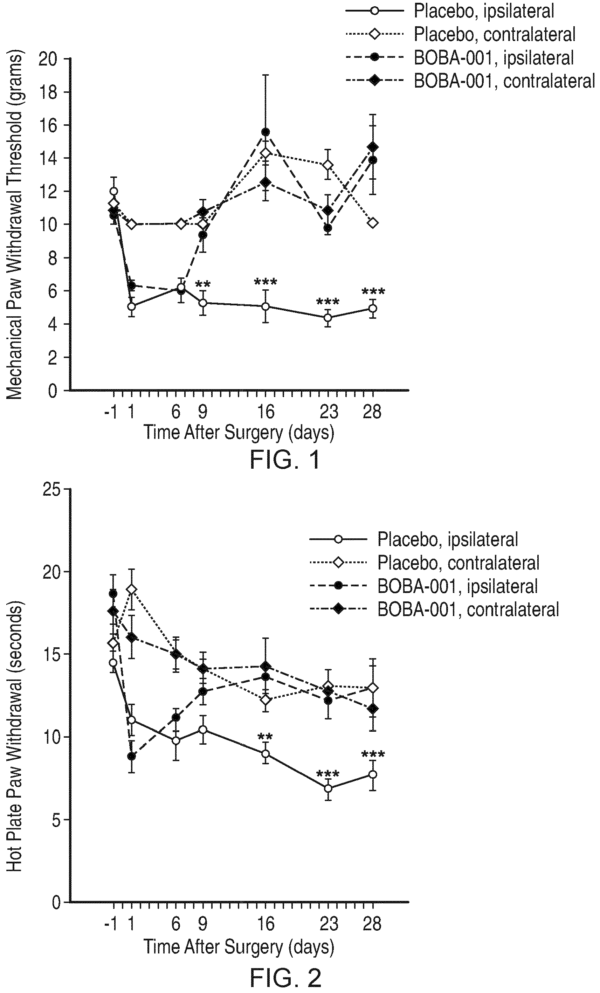

Figure 1 is a graph of mechanical paw withdrawal threshold (grams) versus time after surgery (days), for (a) treatment with placebo, ipsilateral to peripheral nerve injury (open circles); (b) treatment with placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.
Figure 2 is a graph of hot plate paw withdrawal (seconds) versus time after surgery (days), for (a) treatment with placebo, ipsilateral to peripheral nerve injury (open circles); (b) treatment with placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.

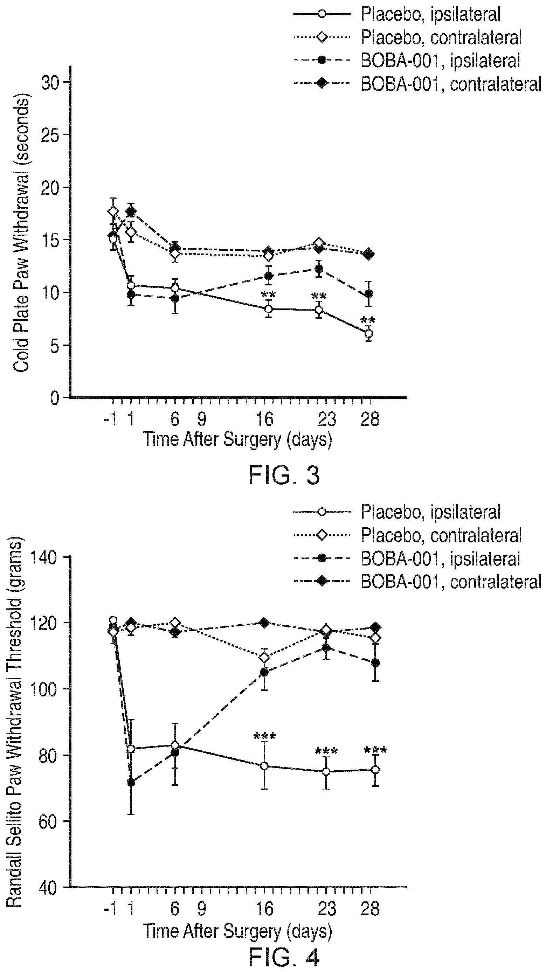

Figure 3 is a graph of cold plate paw withdrawal (seconds) versus time after surgery (days), for (a) treatment with placebo, ipsilateral to peripheral nerve injury (open circles); (b) placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.
Figure 4 is a graph of Randall Sellito paw withdrawal threshold (grams) versus time after surgery (days), for (a) treatment with placebo, ipsilateral to peripheral nerve injury (open circles); (b) placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.

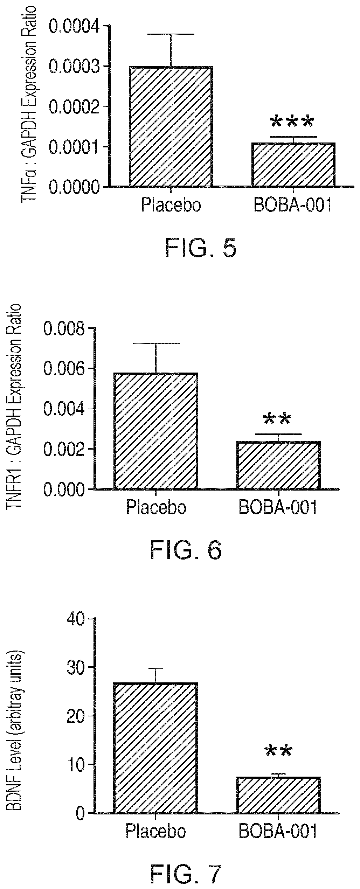

Figure 5 is a bar graph showing the TNFα : GAPDH expression ratio in the dorsal horn ipsilateral to peripheral nerve injury for (a) rats treated with placebo and (b) rats treated with BOBA-001. Three animals per group (n=3). The data are reported as mean ± standard error of mean (SEM); Student's t-test: ***p* ≤0.01, ****p* ≤0.001.
Figure 6 is a bar graph showing the TNFR1 : GAPDH expression ratio in the dorsal horn ipsilateral to peripheral nerve injury for (a) rats treated with placebo and (b) rats treated with BOBA-001. Three animals per group (n=3). The data are reported as mean ± standard error of mean (SEM); Student's t-test: ***p* ≤0.01, ****p* ≤0.001.
Figure 7 is a bar graph showing the BDNF level (arbitray units) in the dorsal horn ipsilateral to peripheral nerve injury for (a) rats treated with placebo and (b) rats treated with BOBA-001. Five serial sections per injured cord analysed, from 3 animals per group (n=3). The data are reported as mean ± standard error of mean (SEM); Student's t-test: ***p* ≤0.01, ****p* ≤0.001.

### DETAILED DESCRIPTION OF THE INVENTION

### Compounds

The present invention relates to certain compounds, and pharmaceutical compositions comprising those compounds, which are useful for the treatment or prevention of neuropathic pain, as described herein.

The compounds are related to 4-[5-(benzofuran-2-yl)-1,2,4-oxadiazol-3-yl]benzoic acid, shown below:

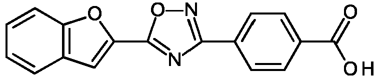

Accordingly, the compounds are compounds of the following formula, and pharmaceutically acceptable salts, hydrates, and solvates thereof, wherein -R^{Q1}, -R^{Q2}, -R^{Q3}, -R^{Q4}, -R^{Q5}, -R^{P1} -R^{P2}, and -R^{A} are as defined herein (for convenience, collectively referred to herein as "benzofuranyl-oxadiazolyl-benzoic acid compounds" or "BOBA compounds"):

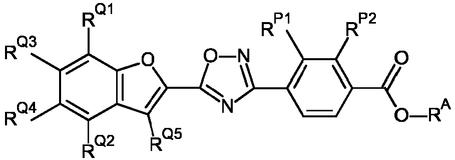

In some embodiments of the invention, the compounds are defined as follows:
(1) A compound of the following formula, or a pharmaceutically acceptable salt, hydrate, or solvate thereof: 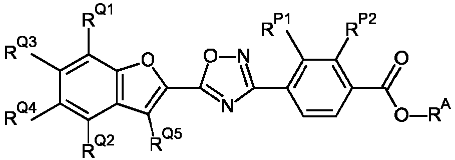 wherein:
   -R^{Q1} is independently -R^{Q1A}, -R^{Q1B}, or -R^{Q1C};
   -R^{Q1A} is saturated linear or branched C₁₋₄alkyl;
   -R^{Q1B} is independently -F, -Cl, -Br, or -I;
   -R^{Q1C} is -CF₃;
   -R^{Q2} is independently -R^{Q2A}, -R^{Q2B}, or -R^{Q2C};
   -R^{Q2A} is saturated linear or branched C₁₋₄alkyl;
   -R^{Q2B} is independently -F, -Cl, -Br, or -I;
   -R^{Q2C} is -CF₃;
   -R^{Q3} is independently -H, -R^{Q3A}, -R^{Q3B}, or -R^{Q3C};
   -R^{Q3A} is saturated linear or branched C₁₋₄alkyl;
   -R^{Q3B} is independently -F, -Cl, -Br, or -I;
   -R^{Q3C} is -CF₃;
   -R^{Q4} is independently -H, -R^{Q4A}, -R^{Q4B}, or -R^{Q4C};
   -R^{Q4A} is saturated linear or branched C₁₋₄alkyl;
   -R^{Q4B} is independently -F, -Cl, -Br, or -I;
   -R^{Q4C} is -CF₃;
   -R^{Q5} is independently -H, -R^{Q5A}, -R^{Q5B}, or -R^{Q5C};
   -R^{Q5A} is saturated linear or branched C₁₋₄alkyl;
   -R^{Q5B} is independently -F, -Cl, -Br, or -I;
   -R^{Q5C} is -CF₃;
   -R^{P1} is independently -H or -R^{P1X};
   -R^{P1X} is independently -R^{P1A}, -R^{P1B}, -R^{P1C}, or -R^{P1D};
   -R^{P1A} is saturated linear or branched C₁₋₄alkyl;
   -R^{P1B} is independently -F, -Cl, -Br, or -I;
   -R^{P1C} is -CF₃;
   -R^{P1D} is independently -NH₂, -NHR^{P1DD}, or -NR^{P1DD}₂;
   -R^{P1DD} is saturated linear or branched C₁₋₄alkyl;
   -R^{P2} is independently -H or -R^{P2X};
   -R^{P2X} is independently -R^{P2A}, -R^{P2B}, -R^{P2C}, or -R^{P2D};
   -R^{P2A} is saturated linear or branched C₁₋₄alkyl;
   -R^{P2B} is independently -F, -Cl, -Br, or -I;
   -R^{P2C} is -CF₃;
   -R^{P2D} is independently -NH₂, -NHR^{P2DD}, or -NR^{P2DD}₂;
   -R^{P2DD} is saturated linear or branched C₁₋₄alkyl;
   -R^{A} is independently -H or -R^{AA}; and
   -R^{AA} is independently saturated linear or branched C₁₋₄alkyl, phenyl, or benzyl.

The term "saturated linear or branched C₁₋₃alkyl" means -CH₃ (methyl, -Me), -CH₂CH₃ (ethyl, -Et), -CH₂CH₂CH₃ (n-propyl, -nPr), and -CH(CH₃)₂ (iso-propyl, -iPr).

The term "saturated linear or branched C₁₋₄alkyl" further includes -CH₂CH₂CH₂CH₃ (n-butyl, -nBu), -CH₂CH(CH₃)₂ (iso-butyl, -iBu), -CH(CH₃)CH₂CH₃ (sec-butyl, -sBu), and -CH(CH₃)₃ (tert-butyl, -tBu).

For the avoidance of doubt, it is not intended that any two or more of -R^{Q1}, -R^{Q2}, -R^{Q3}, -R^{Q4}, -R^{Q5}, -R^{P1} R^{P2}, and -R^{A} together form a ring fused to the ring (or rings) to which they are attached. For example, it is not intended that -R^{P1} and -R^{P2} together form a ring fused to the ring to which they are attached. Similarly, it is not intended that -R^{Q1} and -R^{Q3} together form a ring fused to the ring to which they are attached.

### The Group -R^{Q1}

(2) A compound according to (1), wherein -R^{Q1} is -R^{Q1A}.
(3) A compound according to (1), wherein -R^{Q1} is -R^{Q1B}.
(4) A compound according to (1), wherein -R^{Q1} is -R^{Q1C}.

### The Group -R^{Q2}

(5) A compound according to any one of (1) to (4), wherein -R^{Q2} is -R^{Q2A}.
(6) A compound according to any one of (1) to (4), wherein -R^{Q2} is -R^{Q2B}.
(7) A compound according to any one of (1) to (4), wherein -R^{Q2} is -R^{Q2C}.

### The Group -R^{Q3}

(8) A compound according to any one of (1) to (7), wherein -R^{Q3} is -H.
(9) A compound according to any one of (1) to (7), wherein -R^{Q3} is -R^{Q3A}.
(10) A compound according to any one of (1) to (7), wherein -R^{Q3} is -R^{Q3B}.
(11) A compound according to any one of (1) to (7), wherein -R^{Q3} is -R^{Q3C}.

### The Group -R^{Q4}

(12) A compound according to any one of (1) to (11), wherein -R^{Q4} is -H.
(13) A compound according to any one of (1) to (11), wherein -R^{Q4} is -R^{Q4A}.
(14) A compound according to any one of (1) to (11), wherein -R^{Q4} is -R^{Q4B}.
(15) A compound according to any one of (1) to (11), wherein -R^{Q4} is -R^{Q4C}.

### The Group -R^{Q5}

(16) A compound according to any one of (1) to (15), wherein -R^{Q5} is -H.
(17) A compound according to any one of (1) to (15), wherein -R^{Q5} is -R^{Q5A}.
(18) A compound according to any one of (1) to (15), wherein -R^{Q5} is -R^{Q5B}.
(19) A compound according to any one of (1) to (15), wherein -R^{Q5} is -R^{Q5C}.

### The Group -R^{Q1A}

(20) A compound according to any one of (1) to (19), wherein -R^{Q1A}, if present, is saturated linear or branched C₁₋₃alkyl.
(21) A compound according to any one of (1) to (19), wherein -R^{Q1A}, if present, is -Me.

### The Group -R^{Q2A}

(22) A compound according to any one of (1) to (21), wherein -R^{Q2A}, if present, is saturated linear or branched C₁₋₃alkyl.
(23) A compound according to any one of (1) to (21), wherein -R^{Q2A}, if present, is -Me.

### The Group -R^{Q3A}

(24) A compound according to any one of (1) to (23), wherein -R^{Q3A}, if present, is saturated linear or branched C₁₋₃alkyl.
(25) A compound according to any one of (1) to (23), wherein -R^{Q3A}, if present, is -Me.

### The Group -R^{Q4A}

(26) A compound according to any one of (1) to (25), wherein -R^{Q4A}, if present, is saturated linear or branched C₁₋₃alkyl.
(27) A compound according to any one of (1) to (25), wherein -R^{Q4A}, if present, is -Me.

### The Group -R^{Q5A}

(28) A compound according to any one of (1) to (27), wherein -R^{Q5A}, if present, is saturated linear or branched C₁₋₃alkyl.
(29) A compound according to any one of (1) to (27), wherein -R^{Q5A}, if present, is -Me.

### The Group -R^{Q1B}

(30) A compound according to any one of (1) to (29), wherein -R^{Q1B}, if present, is independently -F or -Cl.
(31) A compound according to any one of (1) to (29), wherein -R^{Q1B}, if present, is -F.
(32) A compound according to any one of (1) to (29), wherein -R^{Q1B}, if present, is -Cl.

### The Group -R^{Q2B}

(33) A compound according to any one of (1) to (32), wherein -R^{Q2B}, if present, is independently -F or -Cl.
(34) A compound according to any one of (1) to (32), wherein -R^{Q2B}, if present, is -F.
(35) A compound according to any one of (1) to (32), wherein -R^{Q2B}, if present, is -Cl.

### The Group -R^{Q3B}

(36) A compound according to any one of (1) to (35), wherein -R^{Q3B}, if present, is independently -F or -Cl.
(37) A compound according to any one of (1) to (35), wherein -R^{Q3B}, if present, is -F.
(38) A compound according to any one of (1) to (35), wherein -R^{Q3B}, if present, is -Cl.

### The Group -R^{Q4B}

(39) A compound according to any one of (1) to (38), wherein -R^{Q4B}, if present, is independently -F or -Cl.
(40) A compound according to any one of (1) to (38), wherein -R^{Q4B}, if present, is -F.
(41) A compound according to any one of (1) to (38), wherein -R^{Q4B}, if present, is -Cl.

### The Group -R^{Q5B}

(42) A compound according to any one of (1) to (42), wherein -R^{Q5B}, if present, is independently -F or -Cl.
(43) A compound according to any one of (1) to (42), wherein -R^{Q5B}, if present, is -F.
(44) A compound according to any one of (1) to (42), wherein -R^{Q5B}, if present, is -Cl.

### The Group -R^{P1}

(45) A compound according to any one of (1) to (44), wherein -R^{P1} is -H.
(46) A compound according to any one of (1) to (44), wherein -R^{P1} is -R^{P1X}.

### The Group -R^{P2}

(47) A compound according to any one of (1) to (46), wherein -R^{P2} is -H.
(48) A compound according to any one of (1) to (46), wherein -R^{P2} is -R^{P2X}.

### The Group -R^{P1X}

(49) A compound according to any one of (1) to (48), wherein -R^{P1X}, if present, is independently -R^{P1A}, -R^{P1B}, or -R^{P1C}.
(50) A compound according to any one of (1) to (48), wherein -R^{P1X}, if present, is -R^{P1A}.
(51) A compound according to any one of (1) to (48), wherein -R^{P1X}, if present, is -R^{P1B}.
(52) A compound according to any one of (1) to (48), wherein -R^{P1X}, if present, is -R^{P1C}.

### The Group -R^{P2X}

(53) A compound according to any one of (1) to (52), wherein -R^{P2X}, if present, is independently -R^{P2A}, -R^{P2B}, or -R^{P2C}.
(54) A compound according to any one of (1) to (52), wherein -R^{P2X}, if present, is -R^{P2A}.
(55) A compound according to any one of (1) to (52), wherein -R^{P2X}, if present, is -R^{P2B}.
(56) A compound according to any one of (1) to (52), wherein -R^{P2X}, if present, is -R^{P2C}.

### The Group -R^{P1A}

(57) A compound according to any one of (1) to (56), wherein -R^{P1A}, if present, is saturated linear or branched C₁₋₃alkyl.
(58) A compound according to any one of (1) to (56), wherein -R^{P1A}, if present, is -Me.

### The Group -R^{P2A}

(59) A compound according to any one of (1) to (58), wherein -R^{P2A}, if present, is saturated linear or branched C₁₋₃alkyl.
(60) A compound according to any one of (1) to (58), wherein -R^{P2A}, if present, is -Me.

### The Group -R^{P1B}

(61) A compound according to any one of (1) to (60), wherein -R^{P1B}, if present, is independently -F, -CI, or -Br.
(62) A compound according to any one of (1) to (60), wherein -R^{P1B}, if present, is independently -F or -Cl.
(63) A compound according to any one of (1) to (60), wherein -R^{P1B}, if present, is -F.
(64) A compound according to any one of (1) to (60), wherein -R^{P1B}, if present, is -Cl.

### The Group -R^{P2B}

(65) A compound according to any one of (1) to (64), wherein -R^{P2B}, if present, is independently -F, -CI, or -Br.
(66) A compound according to any one of (1) to (64), wherein -R^{P2B}, if present, is independently -F or -Cl.
(67) A compound according to any one of (1) to (64), wherein -R^{P2B}, if present, is -F.
(68) A compound according to any one of (1) to (64), wherein -R^{P2B}, if present, is -Cl.

### The Group -R^{P1D}

(69) A compound according to any one of (1) to (68), wherein -R^{P1D}, if present, is -NH₂.

### The Group -R^{P2D}

(70) A compound according to any one of (1) to (69), wherein -R^{P2D}, if present, is -NH₂.

### The Group -R^{P1DD}

(71) A compound according to any one of (1) to (70), wherein -R^{P1DD}, if present, is saturated linear or branched C₁₋₃alkyl.
(72) A compound according to any one of (1) to (70), wherein -R^{P1DD}, if present, is -Me.

### The Group -R^{P2DD}

(73) A compound according to any one of (1) to (72), wherein -R^{P2DD}, if present, is saturated linear or branched C₁₋₃alkyl.
(74) A compound according to any one of (1) to (72), wherein -R^{P2DD}, if present, is -Me.

### The Group -R^{A}

(75) A compound according to any one of (1) to (74), wherein -R^{A} is -H.
(76) A compound according to any one of (1) to (74), wherein -R^{A} is -R^{AA}.

### The Group -R^{AA}

(77) A compound according to any one of (1) to (76), wherein -R^{AA}, if present, is saturated linear or branched C₁₋₄alkyl.

### Some Preferred Compounds

(78) A compound according to (1), which is compound one of the following formulae, or a pharmaceutically acceptable salt, hydrate, or solvate thereof:

| Code No. | Structure |
|---|---|
| BOBA-001 | 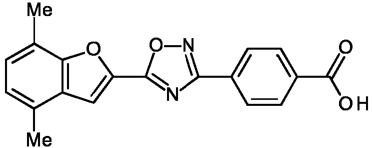 |
| BOBA-002 | 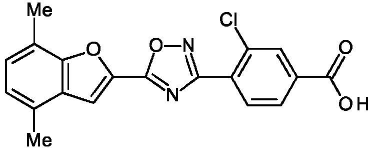 |
| BOBA-003 | 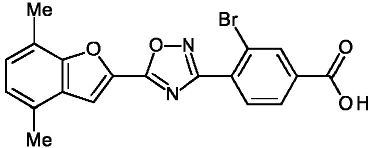 |
| BOBA-004 | 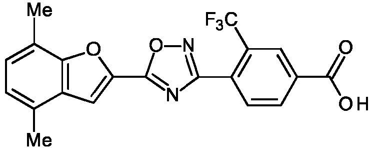 |
| BOBA-005 | 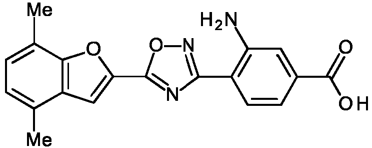 |
| BOBA-006 | 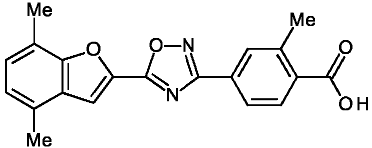 |
| BOBA-007 | 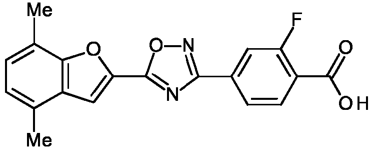 |
| BOBA-008 | 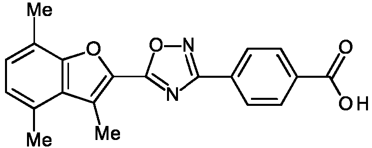 |
| BOBA-009 | 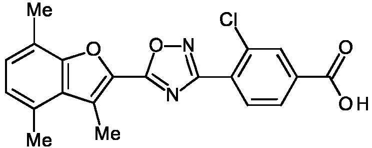 |
| BOBA-010 | 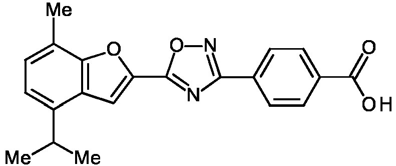 |
| BOBA-011 | 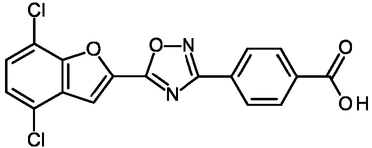 |
| BOBA-012 | 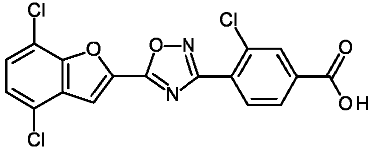 |
| BOBA-013 | 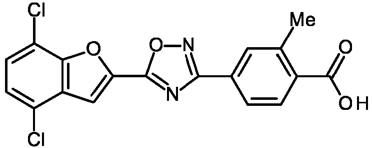 |
| BOBA-014 | 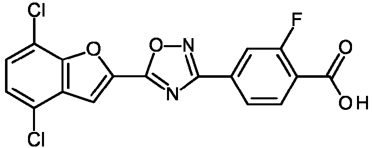 |
| BOBA-015 | 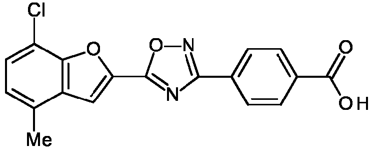 |
| BOBA-016 | 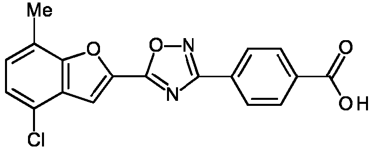 |
| BOBA-017 | 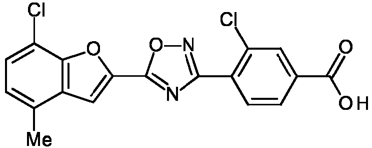 |
| BOBA-018 | 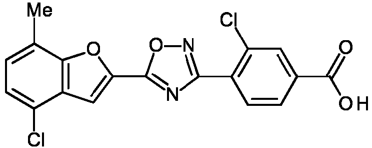 |
| BOBA-019 | 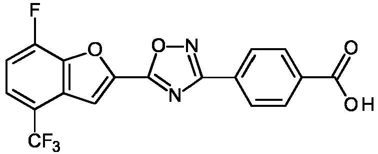 |
| BOBA-020 | 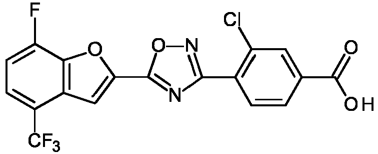 |

(79) A compound according to (1), which is compound of the following formula, or a pharmaceutically acceptable salt, hydrate, or solvate thereof:

| Code No. | Structure |
|---|---|
| BOBA-001 | 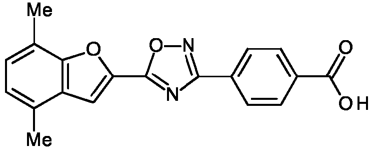 |

### Combinations

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the chemical groups represented by the variables (e.g., -R^{Q1}, -R^{Q1A}, -R^{Q1B}, -R^{Q1C}, -R^{Q2}, -R^{Q2A}, -R^{Q2B}, -R^{Q2C}, -R^{Q3}, -R^{Q3A}, -R^{Q3B}, -R^{Q3C}, -R^{Q4}, -R^{Q4A}, -R^{Q4B}, -R^{Q4C}, -R^{Q5}, -R^{Q5A}, -R^{Q5B}, -R^{Q5C}, -R^{P1}, -R^{P1X}, -R^{P1A}, -R^{P1B}, -R^{P1C}, -R^{P1D}, -R^{P2}, -R^{P2X}, -R^{P2A}, -R^{P2B}, -R^{P2C}, -R^{P2D}, -R^{A}, -R^{AA}, *etc*.) are specifically embraced by the present invention and are disclosed herein just as if each and every combination was individually and explicitly disclosed, to the extent that such combinations embrace compounds that are stable compounds (i.e., compounds that can be isolated, characterised, and tested for biological activity). In addition, all sub-combinations of the chemical groups listed in the embodiments describing such variables are also specifically embraced by the present invention and are disclosed herein just as if each and every such sub-combination of chemical groups was individually and explicitly disclosed herein.

### Substantially Purified Forms

In some embodiments of the invention, the BOBA compounds, as described herein, are used in substantially purified form and/or in a form substantially free from contaminants.

In one embodiment, the compound is in a substantially purified form with a purity of least 50% by weight, e.g., at least 60% by weight, e.g., at least 70% by weight, e.g., at least 80% by weight, e.g., at least 90% by weight, e.g., at least 95% by weight, e.g., at least 97% by weight, e.g., at least 98% by weight, e.g., at least 99% by weight.

In one embodiment, the compound is in a form substantially free from contaminants wherein the contaminants represent no more than 50% by weight, e.g., no more than 40% by weight, e.g., no more than 30% by weight, e.g., no more than 20% by weight, e.g., no more than 10% by weight, e.g., no more than 5% by weight, e.g., no more than 3% by weight, e.g., no more than 2% by weight, e.g., no more than 1% by weight.

### Isomers

Certain compounds may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r-forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

Note that, except as discussed below for tautomeric forms, specifically excluded from the term "isomers," as used herein, are structural (or constitutional) isomers (i.e., isomers which differ in the connections between atoms rather than merely by the position of atoms in space). For example, a reference to a methoxy group, -OCH₃, is not to be construed as a reference to its structural isomer, a hydroxymethyl group, -CH₂OH. However, a reference to a class of structures may well include structurally isomeric forms falling within that class (e.g., C₁₋₄alkyl includes n-propyl and iso-propyl; butyl includes n-, iso-, sec-, and tert-butyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hydroxyazo, and nitro/aci-nitro.

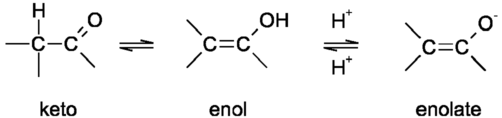

Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Unless otherwise specified, a reference to a particular compound includes all such isomeric forms, including mixtures thereof.

### Salts

It may be convenient or desirable to prepare, purify, and/or handle a corresponding salt of the compound, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19.

For example, if the compound is anionic, or has a functional group which may be anionic (e.g., -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g., -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

Unless otherwise specified, a reference to a particular compound also includes salt forms thereof.

### Hydrates and Solvates

It may be convenient or desirable to prepare, purify, and/or handle a corresponding solvate of the compound. The term "solvate" is used herein in the conventional sense to refer to a complex of solute (e.g., compound, salt of compound) and solvent. If the solvent is water, the solvate may be conveniently referred to as a hydrate, for example, a mono-hydrate, a di-hydrate, a tri-hydrate, *etc.*

Unless otherwise specified, a reference to a particular compound also includes solvate and hydrate forms thereof.

### Chemically Protected Forms

It may be convenient or desirable to prepare, purify, and/or handle the compound in a chemically protected form. The term "chemically protected form" is used herein in the conventional chemical sense and pertains to a compound in which one or more reactive functional groups are protected from undesirable chemical reactions under specified conditions (e.g., pH, temperature, radiation, solvent, and the like). In practice, well known chemical methods are employed to reversibly render unreactive a functional group, which otherwise would be reactive, under specified conditions. In a chemically protected form, one or more reactive functional groups are in the form of a protected or protecting group (also known as a masked or masking group or a blocked or blocking group). By protecting a reactive functional group, reactions involving other unprotected reactive functional groups can be performed, without affecting the protected group; the protecting group may be removed, usually in a subsequent step, without substantially affecting the remainder of the molecule. See, for example, Protective Groups in Organic Synthesis (T. Greene and P. Wuts; 4th Edition; John Wiley and Sons, 2006).

A wide variety of such "protecting," "blocking," or "masking" methods are widely used and well known in organic synthesis. For example, a compound which has two nonequivalent reactive functional groups, both of which would be reactive under specified conditions, may be derivatized to render one of the functional groups "protected," and therefore unreactive, under the specified conditions; so protected, the compound may be used as a reactant which has effectively only one reactive functional group. After the desired reaction (involving the other functional group) is complete, the protected group may be "deprotected" to return it to its original functionality.

For example, an amine group may be protected, for example, as an amide (-NRCO-R) or a urethane (-NRCO-OR), for example, as: a methyl amide (-NHCO-CH₃); a benzyloxy amide (-NHCO-OCH₂C₆H₅, -NH-Cbz); as a t-butoxy amide (-NHCO-OC(CH₃)₃, -NH-Boc); a 2-biphenyl-2-propoxy amide (-NHCO-OC(CH₃)₂C₆H₄C₆H₅, -NH-Bpoc), as a 9-fluorenylmethoxy amide (-NH-Fmoc), as a 6-nitroveratryloxy amide (-NH-Nvoc), as a 2-trimethylsilylethyloxy amide (-NH-Teoc), as a 2,2,2-trichloroethyloxy amide (-NH-Troc), as an allyloxy amide (-NH-Alloc), as a 2(-phenylsulfonyl)ethyloxy amide (-NH-Psec); or, in suitable cases (e.g., cyclic amines), as a nitroxide radical (>N-O●).

For example, a carboxylic acid group may be protected as an ester for example, as: an C₁₋₆alkyl ester (e.g., a methyl ester; a t-butyl ester); a C₁₋₆haloalkyl ester (e.g., a C₁₋₆trihaloalkyl ester); a triC₁₋₆alkylsilyl-C₁₋₇alkyl ester; or a C₅₋₂₀aryl-C₁₋₆alkyl ester (e.g., a benzyl ester; a nitrobenzyl ester); or as an amide, for example, as a methyl amide.

### Prodrugs

It may be convenient or desirable to prepare, purify, and/or handle the compound in the form of a prodrug. The term "prodrug," as used herein, pertains to a compound which, when metabolised (e.g., *in vivo*), yields the desired active compound. Typically, the prodrug is inactive, or less active than the desired active compound, but may provide advantageous handling, administration, or metabolic properties.

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required.

Also, some prodrugs are activated enzymatically to yield the active compound, or a compound which, upon further chemical reaction, yields the active compound (for example, as in ADEPT, GDEPT, LIDEPT, *etc.*)*.* For example, the prodrug may be a sugar derivative or other glycoside conjugate, or may be an amino acid ester derivative.

### General Chemical Synthesis

Methods for the chemical synthesis of the BOBA compounds, as described herein, may be found, for example, in Borthwick *et al.,* 2016. See especially pages 35-48 (general methods) and pages 63-94 (exemplified methods) therein. Additional methods for the chemical synthesis of BOBA-001, as described herein, may be found, for example, in Borthwick *et al.,* 2017. See especially pages 21-28 therein. Those and/or other well-known methods may be modified and/or adapted in known ways in order to facilitate the synthesis of additional BOBA compounds.

### Pharmaceutical Compositions

The pharmaceutical compositions described herein are useful in the treatment or prevention of neuropathic pain.

The pharmaceutical compositions comprise a BOBA compound, as described herein, and a pharmaceutically acceptable carrier, diluent, or excipient.

In one embodiment, the pharmaceutical composition further comprises one or more additional therapeutic agents.

### Uses

The references to the methods of treatment by therapy herein are to be interpreted as references to compounds, pharmaceutical compositions and medicaments for use in those methods.

The BOBA compounds, as described herein, and the pharmaceutical compositions comprising the BOBA compounds, as described herein, are useful in the treatment or prevention of neuropathic pain, as described herein.

One aspect of the invention pertains to a BOBA compound, as described herein, or a pharmaceutical composition comprising a BOBA compound, as described herein, for use in a method of treatment or prevention of neuropathic pain.

Also described herein, but not part of the claimed invention, is use of a BOBA compound, as described herein, in the manufacture of a medicament for treatment or prevention of neuropathic pain. In one embodiment, the medicament comprises the BOBA compound.

Also described herein, but not part of the claimed invention, is a method of treatment or prevention of neuropathic pain, comprising administering to a patient in need of treatment a therapeutically effective amount of a BOBA compound, as described herein, or a pharmaceutical composition comprising a BOBA compound, as described herein.

Also described herein, but not part of the claimed invention, is use of a BOBA compound, as described herein, or a pharmaceutical composition comprising a BOBA compound, as described herein, in the treatment or prevention of neuropathic pain.

### Neuropathic Pain

The present invention relates to certain compounds and pharmaceutical compositions comprising those compounds, which are useful for the treatment or prevention of neuropathic pain, as described herein.

As discussed above, neuropathic pain is a chronic condition caused by a lesion, or pathological change, within the nervous system. Neuropathic pain appears frequently, and often rapidly, after nerve and spinal cord injuries and in certain diseases, producing a debilitation of the patient and a decrease in the quality of life.

Neuropathic pain results from inflammation of the cells within the nervous system, which leads to synapse loss and/or the formation of misconnections in the remaining nerve cells. The BOBA compounds described herein both (a) cause axonal outgrowth, and so can modify synapse connections and (b) have an anti-inflammatory effect. Accordingly, and as demonstrated herein, the compounds can be used in the treatment and prevention of neuropathic pain.

In one embodiment, the neuropathic pain is peripheral neuropathic pain.

In one embodiment, the neuropathic pain is central neuropathic pain.

In one embodiment, the neuropathic pain is chronic neuropathic pain.

In one embodiment, the neuropathic pain is refractory neuropathic pain.

In one embodiment, the neuropathic pain is neuropathic pain associated with a metabolic dysfunction, including, for example, diabetes mellitus and pre-diabetes.

In one embodiment, the neuropathic pain is neuropathic pain associated with diabetes mellitus.

In one embodiment, the neuropathic pain is neuropathic pain associated with pre-diabetes.

In one embodiment, the neuropathic pain is neuropathic pain associated with painful polyneuropathy.

In one embodiment, the neuropathic pain is neuropathic pain associated with painful diabetic neuropathy, including, for example, diabetic peripheral neuropathy.

In one embodiment, the neuropathic pain is neuropathic pain associated with painful diabetic polyneuropathy.

In one embodiment, the neuropathic pain is neuropathic pain associated with post-herpetic neuralgia.

In one embodiment, the neuropathic pain is neuropathic pain associated with trigeminal neuralgia.

In one embodiment, the neuropathic pain is neuropathic pain associated with occipital neuralgia.

In one embodiment, the neuropathic pain is neuropathic pain associated with painful radiculopathy, including, for example, lumbar and cervical painful radiculopathy.

In one embodiment, the neuropathic pain is neuropathic pain associated with an infectious disease, including, for example, herpes zoster (shingles), HIV infection, Lyme disease, diphtheria, and leprosy.

In one embodiment, the neuropathic pain is neuropathic pain associated with a liver or kidney disorder, including, for example, a chronic liver or kidney disorder, including, for example, liver disease, liver failure, kidney disease, and kidney failure.

In one embodiment, the neuropathic pain is neuropathic pain associated with an immune or inflammatory disorder, including, for example, Guillain-Barré syndrome, rheumatoid arthritis, lupus, Sjörgren's syndrome, and coeliac disease.

In one embodiment, the neuropathic pain is neuropathic pain associated with an inherited neuropathy or channelopathy, including, for example, inherited erythromelalgia, paroxysmal extreme pain disorder, and Charcot-Marie-Tooth disease (CMT).

In one embodiment, the neuropathic pain is neuropathic pain associated with small fibre sensory neuropathy.

In one embodiment, the neuropathic pain is neuropathic pain associated with a thyroid hormone disorder, including, for example, hypothyroidism.

In one embodiment, the neuropathic pain is neuropathic pain associated with stroke.

In one embodiment, the neuropathic pain is neuropathic pain associated with cancer, including, for example, lymphoma and multiple myeloma.

In one embodiment, the neuropathic pain is neuropathic pain associated with chemotherapy, for example, cancer chemotherapy.

In one embodiment, the neuropathic pain is neuropathic pain associated with peripheral nerve injury pain.

In one embodiment, the neuropathic pain is neuropathic pain associated with nerve damage following traumatic injury.

In one embodiment, the neuropathic pain is neuropathic pain associated with post-traumatic neuropathy.

In one embodiment, the neuropathic pain is neuropathic pain associated with spinal cord injury, including, for example, spinal cord injury caused by trauma, for example, a road traffic accident.

In one embodiment, the neuropathic pain is neuropathic pain associated with traumatic peripheral nerve injury.

In one embodiment, the neuropathic pain is neuropathic pain associated with post-surgery neuropathy (e.g., post-surgery neuropathic pain).

In one embodiment, the neuropathic pain is neuropathic pain following surgery, including, for example, neuropathic pain following nerve surgery, including, for example, spinal cord surgery.

In one embodiment, the neuropathic pain is neuropathic pain associated with fibromyalgia.

In one embodiment, the neuropathic pain is neuropathic pain associated with lower back pain.

In one embodiment, the neuropathic pain is neuropathic pain associated with carpal tunnel syndrome.

In one embodiment, the neuropathic pain is neuropathic pain associated with causalgia.

In one embodiment, the neuropathic pain is neuropathic pain associated with reflex sympathetic dystrophy (RSD).

In one embodiment, the neuropathic pain is neuropathic pain associated with Complex Regional Pain Syndrome (CRPS), including, for example, Type 1 and Type 2.

In one embodiment, the neuropathic pain is neuropathic pain associated with amputation.

In one embodiment, the neuropathic pain is neuropathic pain associated with a neurodegenerative disease, for example, Parkinson's disease.

In one embodiment, the neuropathic pain is neuropathic pain associated with stroke, including, for example, central post-stroke pain.

In one embodiment, the neuropathic pain is neuropathic pain associated with syringomyelia.

In one embodiment, the neuropathic pain is neuropathic pain associated with a demyelinating disease, including, for example, multiple sclerosis, transverse myelitis, and neuromyelitis optica.

In one embodiment, the neuropathic pain is idiopathic neuropathic pain.

### Treatment and Prevention

As discussed above, the present invention relates to certain compounds and pharmaceutical compositions comprising those compounds, which are useful for the treatment or prevention of neuropathic pain, as described herein.

The compounds may be administered, for example, at or shortly after the time of nerve injury (e.g., following road traffic accident, spinal cord surgery, *etc.*) to prevent or mitigate the development of neuropathic pain. Alternatively, the compounds may be administered when neuropathic pain is detected, for example, during the course of disease (e.g., diabetic neuropathy) or during recovery from nerve injury or surgery.

The term "treatment or prevention," as used herein in the context of treating or preventing a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, alleviation of symptoms of the condition, amelioration of the condition, and cure of the condition. Treatment as a prophylactic measure (*i.e.,* prevention) is also included, for example, use with patients who have not yet developed the condition, but who are at risk of developing the condition.

For example, treatment or prevention of neuropathic pain includes: preventing the onset of neuropathic pain, inhibiting the progress of neuropathic pain, reducing the rate of progress of neuropathic pain, reducing the incidence of neuropathic pain, reducing the severity of neuropathic pain, alleviating one or more symptoms of neuropathic pain, amelioration of neuropathic pain, cure of neuropathic pain, *etc.*

The term "therapeutically-effective amount," as used herein, pertains to that amount of a compound, or a material, composition or dosage form comprising a compound, which is effective for producing some desired therapeutic effect, commensurate with a reasonable benefit/risk ratio, when administered in accordance with a desired treatment regimen.

### Kits

Also described herein, but not part of the claimed invention, is a kit comprising (a) a BOBA compound as described herein, or a composition comprising a BOBA compound, as described herein, *e.g*., preferably provided in a suitable container and/or with suitable packaging; and (b) instructions for use, *e.g*., written instructions on how to administer the compound or composition, for the treatment or prevention of neuropathic pain.

### Routes of Administration

The BOBA compound or pharmaceutical composition comprising the BOBA compound may be administered to a subject by any convenient route of administration, whether systemically/peripherally or topically (*i.e.,* at the site of desired action).

Routes of administration include oral (*e.g*., by ingestion); buccal; sublingual; transdermal (including, *e.g.,* by a patch, plaster, *etc.*); transmucosal (including, *e.g.,* by a patch, plaster, *etc.*); intranasal (*e.g.,* by nasal spray, drops or from an atomiser or dry powder delivery device); ocular *(e.g.,* by eyedrops); pulmonary *(e.g.,* by inhalation or insufflation therapy using, *e.g.,* an aerosol, *e.g.,* through the mouth or nose); rectal *(e.g.,* by suppository or enema); vaginal (*e.g*., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

In one preferred embodiment, the route of administration is oral (*e.g*., by ingestion).

### The Subject/Patient

The subject/patient may be a chordate, a vertebrate, a mammal, a placental mammal, a marsupial *(e.g.,* kangaroo, wombat), a rodent (*e.g.,* a guinea pig, a hamster, a rat, a mouse), murine (*e.g.,* a mouse), a lagomorph (*e.g.,* a rabbit), avian (*e.g.,* a bird), canine (*e.g*., a dog), feline (*e.g.*, a cat), equine (*e.g*., a horse), porcine (*e.g*., a pig), ovine (*e.g*., a sheep), bovine (*e.g.,* a cow), a primate, simian (*e.g.,* a monkey or ape), a monkey (*e.g.,* marmoset, baboon), an ape (*e.g.,* gorilla, chimpanzee, orangutan, gibbon), or a human. Furthermore, the subject/patient may be any of its forms of development, for example, a foetus.

In one preferred embodiment, the subject/patient is a human.

### Formulations

While it is possible for the BOBA compound to be administered alone, it is preferable to present it as a pharmaceutical composition (*e.g*., formulation, preparation, medicament) comprising at least one BOBA compound, as described herein, together with one or more other pharmaceutically acceptable ingredients well known to those skilled in the art, including pharmaceutically acceptable carriers, diluents, excipients, adjuvants, fillers, buffers, preservatives, anti-oxidants, lubricants, stabilisers, solubilisers, surfactants (*e.g*., wetting agents), masking agents, colouring agents, flavouring agents, and sweetening agents. The composition may further comprise other active agents, for example, other therapeutic or prophylactic agents.

Also described herein, but not part of the claimed invention, are methods of making a pharmaceutical composition comprising mixing at least one BOBA compound, as described herein, together with one or more other pharmaceutically acceptable ingredients well known to those skilled in the art, *e.g.,* carriers, diluents, excipients, *etc.* If formulated as discrete units (*e.g.,* tablets, *etc.*)*,* each unit contains a predetermined amount (dosage) of the compound.

The term "pharmaceutically acceptable," as used herein, pertains to compounds, ingredients, materials, compositions, dosage forms, *etc.,* which are, within the scope of sound medical judgment, suitable for use in contact with the tissues of the subject in question (*e.g*., human) without excessive toxicity, irritation, allergic response, or other problem or complication, commensurate with a reasonable benefit/risk ratio. Each carrier, diluent, excipient, *etc.* must also be "acceptable" in the sense of being compatible with the other ingredients of the formulation.

Suitable carriers, diluents, excipients, *etc.* can be found in standard pharmaceutical texts, for example, Remington's Pharmaceutical Sciences, 18th edition, Mack Publishing Company, Easton, Pa., 1990; and Handbook of Pharmaceutical Excipients, 5th edition, 2005.

The formulations may be prepared by any methods well known in the art of pharmacy. Such methods include the step of bringing into association the compound with a carrier which constitutes one or more accessory ingredients. In general, the formulations are prepared by uniformly and intimately bringing into association the compound with carriers (*e.g.,* liquid carriers, finely divided solid carrier, *etc.*), and then shaping the product, if necessary.

The formulation may be prepared to provide for rapid or slow release; immediate, delayed, timed, or sustained release; or a combination thereof.

Formulations may suitably be in the form of liquids, solutions (*e.g*., aqueous, non-aqueous), suspensions (*e.g.,* aqueous, non-aqueous), emulsions (*e.g.,* oil-in-water, water-in-oil), elixirs, syrups, electuaries, mouthwashes, drops, tablets (including, *e.g*., coated tablets), granules, powders, lozenges, pastilles, capsules (including, *e.g*., hard and soft gelatin capsules), cachets, pills, ampoules, boluses, suppositories, pessaries, tinctures, gels, pastes, ointments, creams, lotions, oils, foams, sprays, mists, or aerosols.

Formulations may suitably be provided as a patch, adhesive plaster, bandage, dressing, or the like which is impregnated with one or more compounds and optionally one or more other pharmaceutically acceptable ingredients, including, for example, penetration, permeation, and absorption enhancers. Formulations may also suitably be provided in the form of a depot or reservoir.

The compound may be dissolved in, suspended in, or mixed with one or more other pharmaceutically acceptable ingredients. The compound may be presented in a liposome or other microparticulate which is designed to target the compound, for example, to blood components or one or more organs.

Formulations suitable for oral administration (*e.g*., by ingestion) include liquids, solutions (*e.g.,* aqueous, non-aqueous), suspensions (*e.g.,* aqueous, non-aqueous), emulsions (*e.g*., oil-in-water, water-in-oil), elixirs, syrups, electuaries, tablets, granules, powders, capsules, cachets, pills, ampoules, boluses.

Formulations suitable for buccal administration include mouthwashes, lozenges, pastilles, as well as patches, adhesive plasters, depots, and reservoirs. Lozenges typically comprise the compound in a flavoured basis, usually sucrose and acacia or tragacanth. Pastilles typically comprise the compound in an inert matrix, such as gelatin and glycerin, or sucrose and acacia. Mouthwashes typically comprise the compound in a suitable liquid carrier.

Formulations suitable for sublingual administration include tablets, lozenges, pastilles, capsules, and pills.

Formulations suitable for oral transmucosal administration include liquids, solutions *(e.g.,* aqueous, non-aqueous), suspensions (*e.g.,* aqueous, non-aqueous), emulsions (*e.g.*, oil-in-water, water-in-oil), mouthwashes, lozenges, pastilles, as well as patches, adhesive plasters, depots, and reservoirs.

Formulations suitable for non-oral transmucosal administration include liquids, solutions (*e.g.,* aqueous, non-aqueous), suspensions (*e.g.,* aqueous, non-aqueous), emulsions (*e.g*., oil-in-water, water-in-oil), suppositories, pessaries, gels, pastes, ointments, creams, lotions, oils, as well as patches, adhesive plasters, depots, and reservoirs.

Formulations suitable for transdermal administration include gels, pastes, ointments, creams, lotions, and oils, as well as patches, adhesive plasters, bandages, dressings, depots, and reservoirs.

Tablets may be made by conventional means, *e.g*., compression or moulding, optionally with one or more accessory ingredients. Compressed tablets may be prepared by compressing in a suitable machine the compound in a free-flowing form such as a powder or granules, optionally mixed with one or more binders (*e.g*., povidone, gelatin, acacia, sorbitol, tragacanth, hydroxypropylmethyl cellulose); fillers or diluents (*e.g*., lactose, microcrystalline cellulose, calcium hydrogen phosphate); lubricants (*e.g*., magnesium stearate, talc, silica); disintegrants (*e.g*., sodium starch glycolate, cross-linked povidone, cross-linked sodium carboxymethyl cellulose); surface-active or dispersing or wetting agents (*e.g*., sodium lauryl sulfate); preservatives (*e.g*., methyl p-hydroxybenzoate, propyl p-hydroxybenzoate, sorbic acid); flavours, flavour enhancing agents, and sweeteners. Moulded tablets may be made by moulding in a suitable machine a mixture of the powdered compound moistened with an inert liquid diluent. The tablets may optionally be coated or scored and may be formulated so as to provide slow or controlled release of the compound therein using, for example, hydroxypropylmethyl cellulose in varying proportions to provide the desired release profile. Tablets may optionally be provided with a coating, for example, to affect release, for example an enteric coating, to provide release in parts of the gut other than the stomach.

Ointments are typically prepared from the compound and a paraffinic or a water-miscible ointment base.

Creams are typically prepared from the compound and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the compound through the skin or other affected areas. Examples of such dermal penetration enhancers include dimethylsulfoxide and related analogues.

Emulsions are typically prepared from the compound and an oily phase, which may optionally comprise merely an emulsifier (otherwise known as an emulgent), or it may comprise a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulfate. The choice of suitable oils or fats for the formulation is based on achieving the desired cosmetic properties, since the solubility of the compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for intranasal administration, where the carrier is a liquid, include, for example, nasal spray, nasal drops, or by aerosol administration by nebuliser, include aqueous or oily solutions of the compound.

Formulations suitable for intranasal administration, where the carrier is a solid, include, for example, those presented as a coarse powder having a particle size, for example, in the range of about 20 to about 500 microns which is administered in the manner in which snuff is taken, *i.e.,* by rapid inhalation through the nasal passage from a container of the powder held close up to the nose.

Formulations suitable for pulmonary administration (*e.g*., by inhalation or insufflation therapy) include those presented as an aerosol spray from a pressurised pack, with the use of a suitable propellant, such as dichlorodifluoromethane, trichlorofluoromethane, dichloro-tetrafluoroethane, carbon dioxide, or other suitable gases.

Formulations suitable for ocular administration include eye drops wherein the compound is dissolved or suspended in a suitable carrier, especially an aqueous solvent for the compound.

Formulations suitable for rectal administration may be presented as a suppository with a suitable base comprising, for example, natural or hardened oils, waxes, fats, semi-liquid or liquid polyols, for example, cocoa butter or a salicylate; or as a solution or suspension for treatment by enema.

Formulations suitable for vaginal administration may be presented as pessaries, tampons, creams, gels, pastes, foams or spray formulations containing in addition to the compound, such carriers as are known in the art to be appropriate.

Formulations suitable for parenteral administration (*e.g*., by injection), include aqueous or non-aqueous, isotonic, pyrogen-free, sterile liquids (*e.g*., solutions, suspensions), in which the compound is dissolved, suspended, or otherwise provided (*e.g*., in a liposome or other microparticulate). Such liquids may additionally contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection.

Typically, the concentration of the compound in the liquid is from about 1 ng/mL to about 10 µg/mL, for example, from about 10 ng/mL to about 1 µg/mL. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

### Dosage

It will be appreciated by one of skill in the art that appropriate dosages of the BOBA compounds, and compositions comprising the BOBA compounds, can vary from patient to patient. Determining the optimal dosage will generally involve the balancing of the level of therapeutic benefit against any risk or deleterious side effects. The selected dosage level will depend on a variety of factors including the activity of the particular BOBA compound, the route of administration, the time of administration, the rate of excretion of the BOBA compound, the duration of the treatment, other drugs, compounds, and/or materials used in combination, the severity of the condition, and the species, sex, age, weight, condition, general health, and prior medical history of the patient. The amount of BOBA compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the BOBA compound is in the range of about 10 µg to about 20 mg (more typically about 100 µg to about 10 mg; for example, about 1 mg) per kilogram body weight of the subject per day. Where the compound is a salt, an ester, an amide, a prodrug, or the like, the amount administered is calculated on the basis of the parent compound and so the actual weight to be used is increased proportionately.

### CHEMICAL SYNTHESIS

As discussed above, methods for the chemical synthesis of BOBA compounds, as described herein, may be found, for example, in Borthwick *et al.,* 2016. See especially pages 35-48 (general methods) and pages 63-94 (exemplified methods) therein.

Additional methods for the chemical synthesis of BOBA-001, as described herein, may be found, for example, in Borthwick *et al.,* 2017. See especially pages 21-28 therein.

Those and/or other well-known methods may be modified and/or adapted in known ways in order to facilitate the synthesis of additional BOBA compounds.

For ease of reference, details for the chemical synthesis of BOBA-001 are described below.

One synthetic route for the preparation of BOBA-001 is described in Borthwick *et al.,* 2016, and is shown in Scheme 1 below.

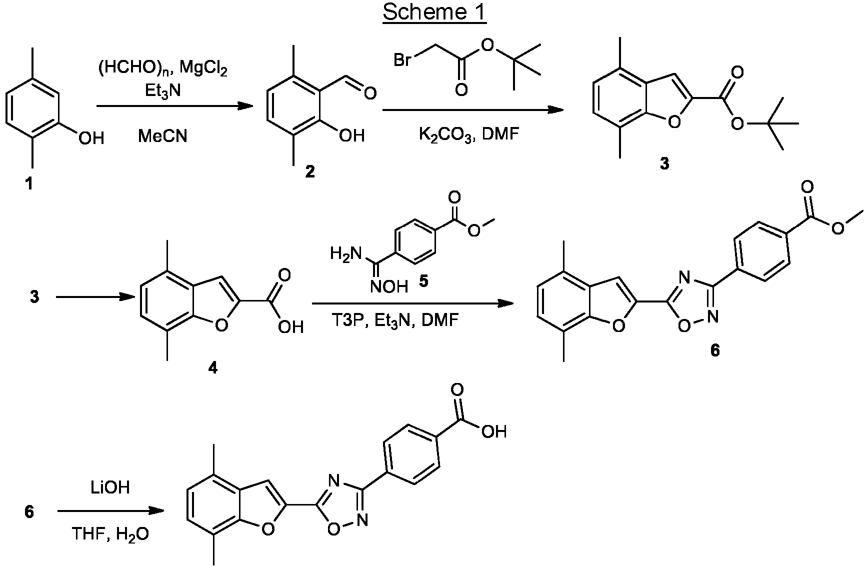

In this approach, 2,5-dimethylphenol **(1)** is reacted with paraformaldehyde to give 2-hydroxy-3,6-dimethylbenzaldehyde **(2),** which is then reacted with *tert-*butyl *2-*bromoacetate to give *tert-*butyl 4,7-dimethylbenzofuran-2-carboxylate **(3),** and deprotected to give 4,7-dimethylbenzofuran-2-carboxylic acid **(4).** This acid is then reacted with methyl 4-(*N*'-hydroxycarbamimidoyl)benzoate **(5)** to give methyl 4-(5-(4,7-dimethylbenzofuran-2-yl)-1,2,4-oxadiazol-3-yl)benzoate **(6),** which is then deprotected to give the target compound, 4-(5-(4,7-dimethylbenzofuran-2-yl)-1,2,4-oxadiazol-3-yl)benzoic acid **(BOBA-001).**

An example of this synthetic route is described below.

### Step 1: 2-Hydroxy-3,6-dimethylbenzaldehyde (2)

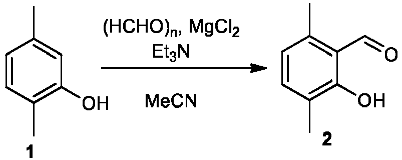

A suspension of 2,5-dimethylphenol **(1)** (20 g, 160 mmol), paraformaldehyde (34 g, 1.1 mol), MgCl₂ (23.4 g, 246 mmol) and Et₃N (86 mL, 610 mmol) in anhydrous MeCN (550 mL) was stirred under reflux for 2 hours. The reaction mixture was concentrated *in vacuo* to half the volume and then partitioned between Et₂O (200 mL) and 1 M HCl (200 mL). The aqueous phase was further extracted with Et₂O (400 mL), then the combined organic extracts were dried over MgSO₄ and filtered. The solution was concentrated *in vacuo* and the residue was purified by silica gel chromatography (330 g, 0-20% Et₂O in isohexane) to afford the title compound **(2)** (8.6 g, 35%) as a yellow solid: ¹H NMR (400 MHz, DMSO-*d₆*) δ: 12.12 (1H, s), 10.29 (1H, s), 7.25 (1H, d), 6.61 (1H, d), 2.56 (3H, s), 2.20 (3H, s).

### Step 2: tert-Butyl 4,7-dimethylbenzofuran-2-carboxylate (3)

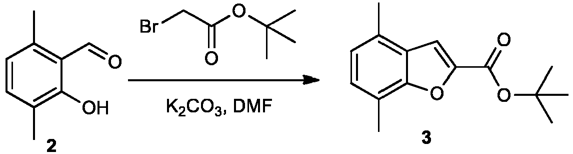

*tert*-Butyl 2-bromoacetate (10.6 mL, 71.5 mmol) was added dropwise to a stirred suspension of 2-hydroxy-3,6-dimethylbenzaldehyde **(2)** (8.6 g, 57 mmol) and potassium carbonate (19.8 g, 143 mmol) in anhydrous DMF (40 mL). The reaction mixture was stirred under reflux for 20 hours. The mixture was partitioned between EtOAc (100 mL) and water (100 mL) and the aqueous phase was further extracted with EtOAc (100 mL). The combined organics were washed with brine (5 x 100 mL) and concentrated *in vacuo.* The residue was purified by silica gel chromatography (330 g, 20% MeOH in dichloromethane (DCM)) to afford the title compound **(3)** (12.3 g, 87% yield) as a red oil: ¹H NMR (400 MHz, DMSO-*d₆*) δ: 7.42 (1H, s), 7.04 (2H, dd), 8.33 (1H, d), 2.52 (3H, s), 2.50 (3H, s), 1.63 (9H, s).

### Step 3: 4,7-Dimethylbenzofuran-2-carboxylic acid (4)

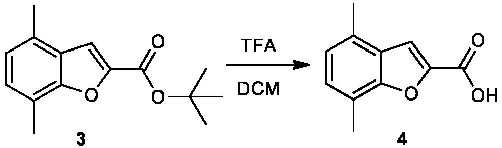

Trifluoroacetic acid (TFA) (19.2 mL, 249 mmol) was added dropwise to a solution of *tert-*butyl 4,7-dimethylbenzofuran-2-carboxylate **(3)** (12.3 g, 49.8 mmol) in DCM (100 mL) at 0°C. After the addition the mixture was allowed to warm to room temperature and stirred for 20 hours. The solvent was removed *in vacuo* and the residue was partitioned between EtOAc (100 mL) and 1 M HCl (100 mL). The aqueous phase was further extracted with EtOAc (100 mL) and the combined organic phases were washed with brine (300 mL) and then concentrated *in vacuo.* The residue was dissolved in EtOAc (100 mL) and then extracted with saturated NaHCO₃ solution (200 mL). The aqueous solution was acidified by the addition of concentrated HCl and extracted with EtOAc (200 mL). The organic solution was concentrated *in vacuo* and co-evaporated with toluene to afford the title compound **(4)** (7.7 g, 81% yield) as a pale brown solid: m/z 190 [M+H]⁺ (ES⁺). ¹H NMR (400 MHz, DMSO-*d₆*) δ: 13.48 (1H, s), 7.72 (1H, s), 7.11 (2H, dd), 2.47 (3H, s), 2.44 (3H, s).

### Step 4: Methyl 4-(5-(4,7-dimethylbenzofuran-2-yl)-1,2,4-oxadiazol-3-yl)benzoate (6)

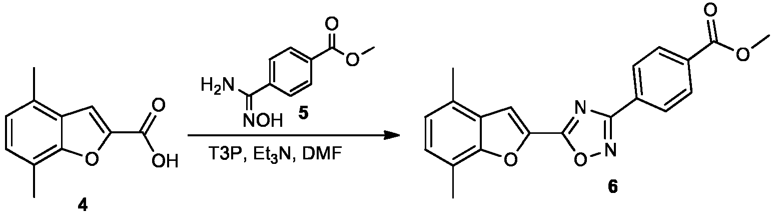

A solution of 2-propanephosphonic acid anhydride (T3P) in EtOAc (50%) (23.2 mL, 39.4 mmol) was added dropwise to a mixture of 4,7-dimethylbenzofuran-2-carboxylic acid **(4)** (3.0 g, 16 mmol), methyl 4-(*N*'-hydroxycarbamimidoyl)benzoate **(5)** (3.1 g, 16 mmol) and Et₃N (11 mL, 79 mmol) in anhydrous N,N-dimethylformamide (DMF) (25 mL) at 0°C. The mixture was stirred at 0°C for 10 minutes then warmed to 90°C and stirred for 18 hours. The reaction mixture was cooled to room temperature and poured into iced water (150 mL). The solid was collected, washed with cold EtOAc and dried under suction. The material was purified by trituration with MeOH and dried *in vacuo* to afford the title compound **(6)** (3.6 g, 65% yield) as a pink solid: m/z 349 [M+H]⁺ (ES⁺). ¹H NMR (400 MHz, DMSO-*d₆*) δ: 8.29-8.26 (3H, m), 8.19 (2H, d), 7.29 (1H, d), 7.13 (1H, d), 3.92 (3H, s), 2.56 (3H, s), 2.54 (3H, s).

### Step 5: 4-(5-(4,7-Dimethylbenzofuran-2-yl)-1,2,4-oxadiazol-3-yl)benzoic acid (BOBA-001)

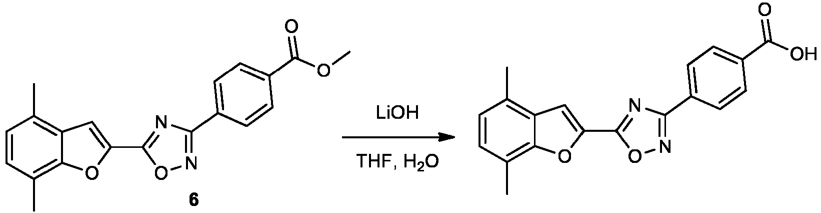

A suspension of methyl 4-(5-(4,7-dimethylbenzofuran-2-yl)-1,2,4-oxadiazol-3-yl)benzoate **(6)** (100 mg, 0.287 mmol) in tetrahydrofuran (THF) (1 mL) was treated with LiOH (2 M, aqueous, 720 µL, 1.4 mmol) and the mixture was stirred at 40°C for 20 hours. The reaction mixture was cooled to room temperature, then acidified by the dropwise addition of 1 M HCl. The resulting solid was collected by filtration, then dissolved in MeOH and evaporated to dryness to afford the title compound (BOBA-001) (95 mg, 99% yield) as a white solid: m/z 335 [M+H]⁺ (ES⁺), 333 [M-H]⁻ (ES). ¹H NMR (400 MHz, DMSO-*d₆*) δ: 13.34 (1H, br. s), 8.25-8.23 (3H, m), 8.16 (2H, d), 7.29 (1H, d), 7.12 (1H, d), 2.56 (3H, s), 2.53 (3H, s).

Another method for the preparation of BOBA-001 is shown in Scheme 2 below.

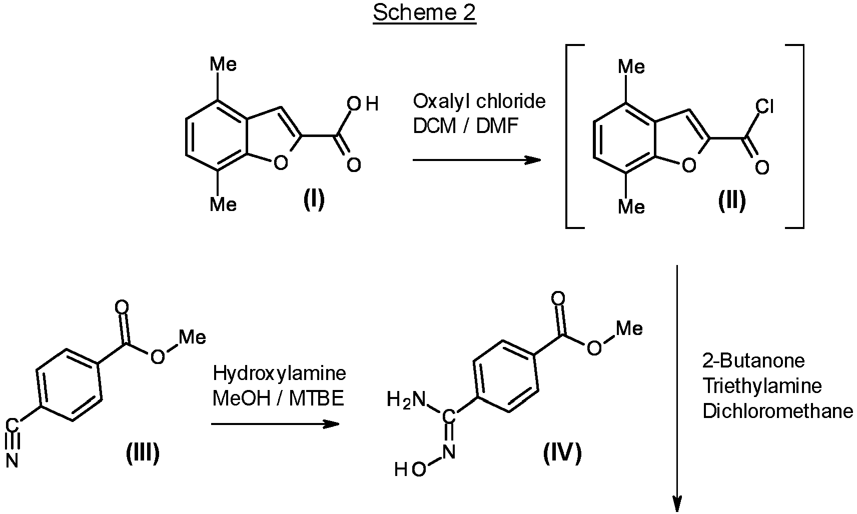

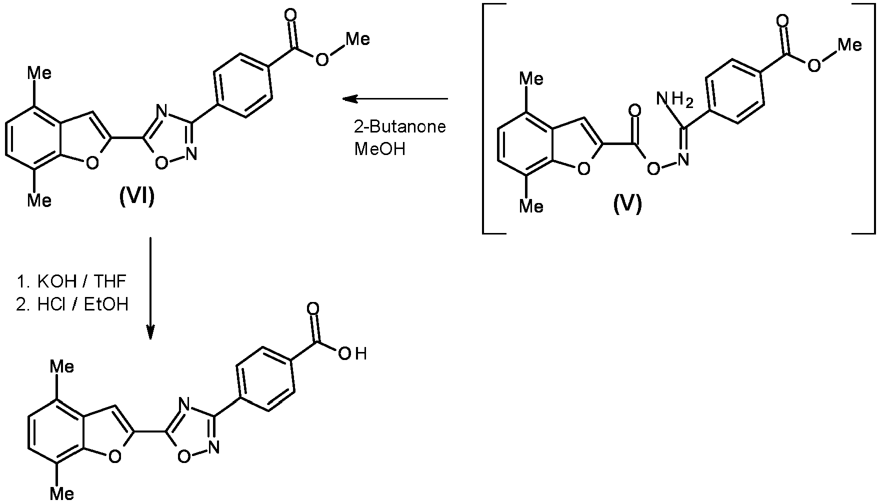

In this approach, methyl 4-cyanobenzoate **(III)** is treated with aqueous hydroxylamine in methanol as solvent. After completion of the reaction, methyl tert-butyl ether (MTBE) is added, the mixture is cooled, and after ageing overnight, the product methyl 4-(N'-hydroxycarbamimidoyl)benzoate **(IV)** is isolated as a solid.

4,7-Dimethylbenzofuran-2-carboxylic acid **(I)** is treated with oxalyl chloride in dichloromethane in the presence of catalytic N,N-dimethylformamide. After completion of the reaction, the solution containing the product 4,7-dimethylbenozfuran-2-carbonyl chloride **(II)** is telescoped to the next stage.

4,7-Dimethylbenozfuran-2-carbonyl chloride **(II)** is treated with methyl 4-(N'-hydroxycarbamimidoyl)benzoate **(IV)** in the presence of triethylamine as base and 2-butanone as solvent. After removal of the solvent the penultimate intermediate methyl 4-[5-(4,7-dimethylbenzofuran-2-yl)-1,2,4-oxadiazol-3-yl]benzoate **(VI)** is crystallised from methanol and isolated by filtration.

Methyl 4-[5-(4,7-dimethylbenzofuran-2-yl)-1,2,4-oxadiazol-3-yl]benzoate **(VI)** is hydrolysed by treatment with potassium hydroxide in tetrahydrofuran (THF) at 60-65°C, and subsequently diluted with water and ethanol. Upon addition of concentrated hydrochloric acid, the target compound 4-[5-(4,7-dimethylbenzofuran-2-yl)-1,2,4-oxadiazol-3-yl]benzoic acid **(BOBA-001)** precipitates and is isolated by filtration.

An example of this synthetic route is described below.

### Step 1: Methyl 4-(N'-hydroxycarbamimidoyl)benzoate (IV)

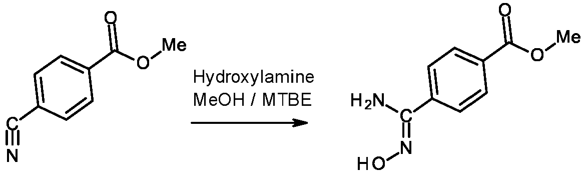

A solution of methyl 4-cyanobenzoate **(III)** (2.50 kg, 1 wt) in methanol (7.5 L, 3 vol) was diluted with methyl tert.-butyl ether (9.3 kg, 3.7 wt). Aqueous hydroxylamine (50% solution; 1.23 kg, 0.5 wt, 1.2 eq.) was dosed over approximately 0.5 hours at 20-25°C. The dosing lines were rinsed with methanol (1 kg, 0.4 wt) and the reaction allowed to proceed for approximately 12 hours at 20-25°C. The batch was cooled to 5 ± 5°C and aged at 5 ± 5°C for 3 hours. The product, methyl 4-(N'-hydroxycarbamimidoyl)benzoate **(IV),** was isolated by filtration, washed with methyl tert.-butyl ether (2 x 5.5 kg, 2 x 2.2 wt), and dried under a flow of nitrogen at ambient for approximately 4 hours (2.38 kg, 93%).

### Step 2: 4,7-Dimethylbenozfuran-2-carbonyl chloride (II)

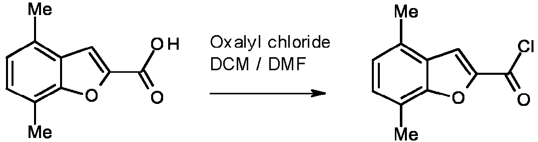

A reactor was charged with 4,7-dimethylbenzofuran-2-carboxylic acid **(I)** (1.96 kg, 1 wt), N,N-dimethylformamide (8 g, 0.004 wt) and dichloromethane (6.85 kg, 3.5 wt) and the batch was heated to 25-30°C. Over a period of about 15 minutes, oxalyl chloride (1.47 kg, 0.75 wt, 1.1 eq) was dosed to the mixture, and the dosing lines were rinsed with dichloromethane (1.3 kg, 0.66 wt). The batch was stirred for approximately 1.5 hours at 30-35°C and the resultant product, 4,7-dimethylbenozfuran-2-carbonyl chloride (II), was used directly in the next step without any further purification.

### Step 3: Methyl 4-[5-(4,7-dimethylbenzofuran-2-yl)-1,2,4-oxadiazol-3-yl]benzoate (VI)

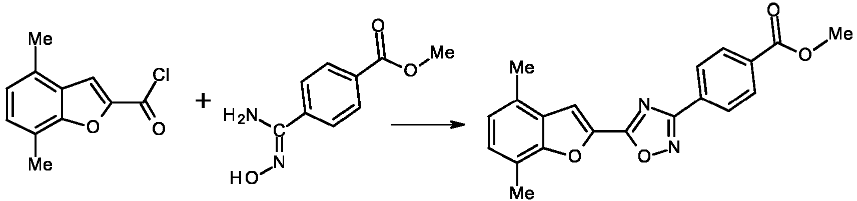

A reactor was charged with methyl 4-(N'-hydroxycarbamimidoyl)benzoate **IV)** (1.97 kg, 1 wt, 0.97 eq based on **(I)),** triethylamine (2.34 kg, 1.2 wt, 2.2 eq) and methyl isobutyl ketone (23.9 kg, 12 wt). The batch was heated to 30-35°C and dosed with the solution of 4,7-dimethylbenozfuran-2-carbonyl chloride **(II)** (prepared in Step 2), ensuring that the temperature did not exceed 60°C. The dosing lines were rinsed with dichloromethane (0.66 wt) and the solvent was initially distilled to remove some solvent, and then heated under atmospheric pressure at 110-115°C for approximately 3 hours. The batch was cooled to 60-70°C, methanol (8 kg, 4 wt) was added, and the batch was heated at this temperature for another 30 minutes. The batch was cooled to 20 ± 3°C and aged at this temperature for approximately 1 hour. The solid product was filtered, washed with methanol (2 x 6.2 kg, 2 x 3.1 wt), and dried under a flow of nitrogen at ambient temperature for 16 hours (3.3 kg, 93%).

### Step 4: 4-[5-(4,7-dimethylbenzofuran-2-yl)-1,2,4-oxadiazol-3-yl]benzoic acid (BOBA-001)

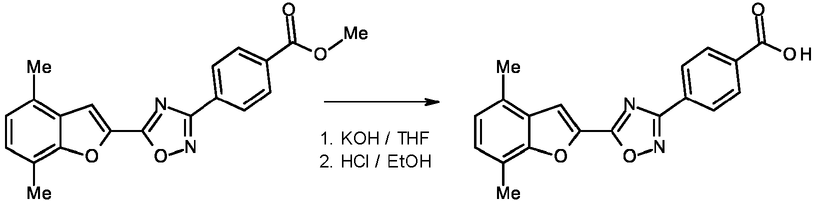

A reactor was charged with methyl 4-[5-(4,7-dimethylbenzofuran-2-yl)-1,2,4-oxadiazol-3-yl]benzoate **(VI)** (3.0 kg, 1 wt), tetrahydrofuran (THF) (22 kg, 7.1 wt) and a solution of aqueous potassium hydroxide, prepared from solid KOH, (0.69 kg, 0.23 wt) in demineralized water (DEMI water, 7.5 kg, 2.5 wt). The batch was heated to 65 ± 5°C and stirred for approximately 3 hours at 65 ± 5°C. The batch was cooled to 30-35°C, and the reaction mixture was discharged. This solution was charged to a reactor via an in-line filter (i.e., a clarification step). The reactor was charged, via an in-line filter, with DEMI water (6 kg, 2.0 wt) and absolute ethanol (9.6 kg, 3.2 wt). The batch was heated to 55 ± 5°C. The reactor was dosed, via an in-line filter, with concentrated (30 wt%) hydrochloric acid (1.35 kg, 0.45 wt) and the batch was stirred for approximately 15 minutes at 55 ± 5°C. The batch was cooled to 20 ± 3°C and aged at this temperature for 2 hours. The product was filtered and the filter cake was washed twice with a mixture of DEMI water (1.5 kg, 0.5 wt) and ethanol absolute (8.4 kg, 2.8 wt). The batch was dried on the filter for approximately 1 hour at ambient temperature to give the target compound, BOBA-001.

### BIOLOGICAL STUDIES

### Animal Studies

### Animals

Adult male Sprague-Dawley rats (150-200 g) were housed under natural light : dark cycles and provided water and food ad lib. They were habituated to the testing paradigms for 3-5 days before data collection. All protocols were approved by the Animal Care and Use Committee of Kings College London and UK Home Office.

### Surgery

Animals were studied using the standard model for neuropathic pain (lumbar 5 spinal nerve ligation, L5 SNL; see, e.g., Kim *et al.,* 1992). This is the most widely used model in neuropathic pain studies because of its high reproducibility of spontaneous and evoked pain (hyperalgesia, allodynia) and the absence of autotomy (see, e.g., Barrot, 2012; Huang *et al.,* 2016).

Adult rats received a partial nerve ligation of the left sciatic nerve (see, e.g., Seltzer *et al.,* 1990) under isoflurane (Abbott Animal Health) inhalation anesthesia. A small incision was made midway up the left thigh to expose the sciatic nerve. The nerve was carefully cleared of surrounding connective tissues at a site near the trochanter just distal to the point at which the posterior biceps semitendinosus nerve branches off the common sciatic nerve. A 5-0 vicryl suture (Ethicon, UK) was inserted into the nerve and tightly ligated so that 1/3 to 1/2 of the nerve thickness was held within the ligature.

### Treatment

Two groups of 11 rats were studied. In each group, the rats recived partial nerve ligation of the left sciatic nerve (L5 SNL), as described above. In one group, the rats were treated with test compound; in the other group, the rats were treated with placebo. In each group, 8 animals were used for behavioral studies (for 28 days following surgery), as described below. In each group, 3 animals were used for tissue analysis (at 14 days following surgery), as described below.

Rats were treated with treatment solution or a placebo, orally, starting on the day after surgery and subsequently every other day for 4 weeks.

A treatment solution was prepared by dissolving 4 mg of test compound (BOBA-001) in 1 mL of 0.1 N aqueous NaHCO₃ (to give a concentration of approximately 4 mg/mL).

For treated animals, treatment solution was administered orally (po), by oral gavage, to give a dose of 3 mg/kg.

For control animals, a corresponding volume of vehicle (0.1 N aqueous NaHCO₃) was administered orally (po), by oral gavage, as placebo.

### Behavioural Studies

Pain thresholds were measured according to the methods described below, baseline at 1 day prior to surgery, and at 1, 6, 9, 16, 23, and 28 days after surgery.

### Pain Threshold - Mechanical Allodynia

Static mechanical withdrawal thresholds were assessed by applying von Frey hairs (Touch Test, Stoelting, IL, USA) to the plantar surface of the hind paw. Unrestrained animals were acclimatized in acrylic cubicles (8 x 5 x 10 cm) on a wire mesh grid for 60 minutes prior to testing. Calibrated von Frey hairs (flexible nylon fibres of increasing diameter that exert defined levels of force) were applied to the plantar surface of the hind paw until the fibre bent. The hair was held in place for 3 seconds or until the paw was withdrawn in a reflex not associated with movement or grooming. Each hair was applied 5 times to both the left paw (ipsilateral to peripheral nerve injury) and the right paw (contralateral to peripheral nerve injury) alternately, starting with the lowest force hair. Hairs of increasing force were applied in sequence, 5 applications per hair. A positive response was recorded when a 40% withdrawal response occurred over 5 applications.

Figure 1 is a graph of mechanical paw withdrawal threshold (grams) versus time after surgery (days), for (a) treatment with placebo, ipsilateral to peripheral nerve injury (open circles); (b) treatment with placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.

### Pain Threshold - Thermal Hyperalgesia - Hot Plate

Thermal hyperalgesia was assessed using a modified method of Hargreaves *et al.,* 1988. Briefly, rats were habituated for 10 minutes to an apparatus consisting of individual Plexiglas boxes on an elevated glass table. A mobile radiant heat source was located under the table and focused on the hind paw, and the paw withdrawal latencies were defined as the time taken by the rat to remove its hind paw from the heat source. The cut-off point was set at 25 seconds to prevent tissue damage. The apparatus was calibrated to give a paw withdrawal latency of approximately 15 seconds in naive rats. For both the left paw (ipsilateral to peripheral nerve injury) and the right paw (contralateral to peripheral nerve injury), an average of three recordings were taken separated by at least 5 minutes.

Figure 2 is a graph of hot plate paw withdrawal (seconds) versus time after surgery (days), for (a) treatment with placebo, ipsilateral to peripheral nerve injury (open circles); (b) treatment with placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.

### Pain Threshold - Thermal Hyperalgesia - Cold Plate

Withdrawal of the hind paw in response to painful cold stimulation was assessed using an Incremental Hot/Cold Plate (IITC Life Sciences) set at 4 ± 0.1 °C. Rats were lightly restrained and the hind paw was held with the plantar surface on the cold plate. The latency to withdraw the paw was measured to the nearest 0.01 seconds. To avoid tissue injury, the maximum latency period permitted was 20 seconds. For both the left paw (ipsilateral to peripheral nerve injury) and the right paw (contralateral to peripheral nerve injury), an average of three recordings were taken separated by at least by at least 5 minutes. The rats were not tested on day 9 following surgery.

Figure 3 is a graph of cold plate paw withdrawal (seconds) versus time after surgery (days), for (a) treatment with placebo, ipsilateral to peripheral nerve injury (open circles); (b) placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.

### Pain Threshold - Randall-Selitto Test

Mechanical nociceptive thresholds were evaluated using an Analgesy-Meter (Ugo Basile, Comerio, Italy) (see, e.g., Raghavendra *et al.,* 2003). Rats were gently held and incremental pressure (maximum 25 g) was applied onto the dorsal surface of the hind paw. The pressure required to elicit paw withdrawal, the paw pressure threshold, in grams, was determined. For both the left paw (ipsilateral to peripheral nerve injury) and the right paw (contralateral to peripheral nerve injury), an average of three recordings were taken separated by at least 5 minutes. The rats were not tested on day 9 following surgery.

Figure 4 is a graph of Randall Sellito paw withdrawal threshold (grams) versus time after surgery (days), for (a) treatment with placebo, ipsilateral to peripheral nerve injury (open circles); (b) placebo, contralateral to peripheral nerve injury (open diamonds); (c) treatment with BOBA-001, ipsilateral to peripheral nerve injury (filled circles); (d) treatment with BOBA-001, contralateral to peripheral nerve injury (filled diamonds). The data are reported as mean ± standard error of mean (SEM); 8 animals per group (n=8); 2-way repeated measures ANOVA: ***p* ≤ 0.01; ****p* ≤ 0.001.

### Results

The data demonstrate that rats subjected to L5 SNL and treated for 4 weeks with BOBA-001 showed significant improvement in thermal and mechanical threshold tests and enjoyed a significant amelioration of pain, as compared to placebo treated rats.

The data demonstrate that treatment with BOBA-001 reduces mechanical and thermal hypersensitivity, and restores mechanical and thermal sensitity, after nerve injury. This is surprising and unexpected in view of the fact that BOBA-001 also causes axonal outgrowth.

### Tissue Analysis

The levels of markers associated with neuropathic pain in the dorsal horn and dorsal root ganglia, ipsilateral and contra to the peripheral nerve injury, were measured: (1) tumour necrosis factor-α (TNF-α), which is implicated in the initiation of NP (see, e.g., Schafers *et al.,* 2003A); (2) TNFR1, which is upregulated after SNL (see, e.g., Schafers *et al.,* 2003B); and (3) brain-derived neurotrophic factor (BDNF) which, apart from its actions on pre- and post-synaptic inhibition (see, e.g., Meacham *et al.,* 2017), is involved in the central sensitization and synaptic plasticity in the Schaffer collateral (SC) and has been shown to contribute to the development and maintenance of neuropathic pain by activation of the dorsal horn NR2B-containing NMDA (NMDA-2B) receptors (see, e.g., Geng *et al.,* 2010).

### Total RNA Isolation and RT-qPCR Analysis

Fourteen days after injury, three rats from each group were sacrificed. Spinal cord was isolated from the area of injury and lysed with Teflon-glass homogenisers and QlAshredder columns (Qiagen). Total RNA was isolated with the Qiagen RNeasy^{®} Mini kit and reverse transcribed with a QuantiTect^{®} Reverse Transcription kit (Qiagen).

Levels of tumour necrosis factor α (TNFα) and tumour necrosis factor receptor 1 (TNFR1) expression were quantitated in triplicate, relative to glyceraldehyde 3-phosphate dehydrogenase (GAPDH) and triplicate non-template controls for each gene, by RT-qPCR analysis on a LightCycler^{®} 480 instrument with LightCycler^{®} SYBR^{®} Green I Master reagent (Roche).

Primer pairs used were:
TNFα forward: 5'-CTGTGCCTCAGCCTCTTCTC-3';
TNFα reverse: 5'-ACTGATGAGAGGGAGCCCAT-3';
TNFR1 forward: 5'-CCTCTCTCCCCTCGGCTTTA-3';
TNFR1 reverse: 5'-CCCGGGTTAGAAAGGCTCAA-3';
GAPDH forward: 5'-TGACCTCAACTACATGGTCTACA-3'; and
GAPDH reverse: 5'-GACTCCACGACATACTCAGCA-3'.

RT-qPCR cycling parameters were optimised for each gene and were as follows:
45 cycles of 95 °C, 10 seconds; 60 °C, 20 seconds; 72 °C, 10 seconds, following an initial step of 95 °C, 5 minutes.

Figure 5 is a bar graph showing the TNFα : GAPDH expression ratio in the dorsal horn ipsilateral to peripheral nerve injury for (a) rats treated with placebo and (b) rats treated with BOBA-001. Three animals per group (n=3). The data are reported as mean ± standard error of mean (SEM); Student's t-test: ***p* ≤0.01, ****p* ≤0.001.

Figure 6 is a bar graph showing the TNFR1 : GAPDH expression ratio in the dorsal horn ipsilateral to peripheral nerve injury for (a) rats treated with placebo and (b) rats treated with BOBA-001. Three animals per group (n=3). The data are reported as mean ± standard error of mean (SEM); Student's t-test: ***p* ≤0.01, ****p* ≤0.001.

### Immunocytochemistry (IHC)

Spinal cord tissue embedded in paraffin wax was first dewaxed in xylene and 100% industrial methylated spirit (IMS), then heated in citric acid (10 mM, pH 6), until boiling, and then washed under running tap water for 5 minutes. The sections were washed 3x for 5 minutes each in phosphate buffered saline (PBS) before incubation with primary antibody in PBS-0.02% Tween at 4 °C overnight. Primary antibody was removed by washing 3x for 5 minutes each in PBS. Samples were then incubated in the secondary antibody for 1 hour at room temperature in PBS-0.02% Tween, and then washed 3x for 5 minutes each in PBS. The antibodies used were: BDNF antibody (Novuf Biologicals, NBP1-46750) and AlexaFluor^{™} 594.

Multichannel fluorescence (DAPI-FITC-Texas Red filter set) images were captured using a Zeiss LSM 700 laser-scanning confocal microscope. For high magnification images, a 63 x oil-immersion Aprochromat objective (Carl Zeiss) was used. Settings for gain, aperture, contrast, and brightness were optimized initially, and held constant throughout each study so that all sections were digitized under the same conditions of illumination. Channels were imaged sequentially to eliminate bleed-through and multichannel image overlays were obtained using Adobe Photoshop 7.0 (Adobe Systems). Axiovision software was used to collect information on pixel immunoreactivity used for quantitative purposes. All statistical analysis was performed using SigmaSTAT software (SPSS Software Ltd, Birmingham, UK).

Figure 7 is a bar graph showing the BDNF level (arbitray units) in the dorsal horn ipsilateral to peripheral nerve injury for (a) rats treated with placebo and (b) rats treated with BOBA-001. Five serial sections per injured cord analysed, from 3 animals per group (n=3). The data are reported as mean ± standard error of mean (SEM); Student's t-test: ***p* ≤0.01, ****p* ≤0.001.

### Results

The data demonstrate that treatment with BOBA-001 modulates TNF-α, TNFR1, and BDNF after nerve injury. The expression levels of these three markers (TNFα, TNFR1, and BDNF) in the dorsal horn ipsilateral to injury were significantly down-regulated after the treatment with BOBA-001 as compared to placebo. These results strongly suggest a modulatory effect in the pathways that lead to neuropathic pain.

The foregoing has described the principles, preferred embodiments, and modes of operation of the present invention. However, the invention should not be construed as limited to the particular embodiments discussed. Instead, the above-described embodiments should be regarded as illustrative rather than restrictive.

### REFERENCES

A number of publications are cited herein in order to more fully describe and disclose the invention and the state of the art to which the invention pertains. Full citations for these publications are provided below.
Barrot, 2012, "Tests and models of nociception and pain in rodents", Neuroscience, Vol. 211, pp. 39-50.
Borthwick et al., 2016, "Bicycloheteroaryl-heteroaryl-benzoic acid compounds as retinoic acid receptor beta (RARβ) agonists", International Patent Publication No. WO 2016/097004 A1, published 23 June 2016.
Borthwick et al., 2017, "Crystalline forms of 4-(5-(4,7-dimethylbenzofuran-2-yl)-1,2,4-oxadiazol-3-yl)benzoic acid and process for their preparation", International Patent Publication No. WO 2017/220446 A1, published 28 December 2017.
Chen et al., 2014, "Presynaptic GABAergic inhibition regulated by BDNF contributes to neuropathic pain induction", Nat Commun, Vol. 5, p. 5331.
Chiang et al., 2008, "Methods of treating neuropathic pain with retinoic acid receptor agonists", International Patent Publication No. WO 2008/057930 A2, published 15 May 2008.
Colloca et al., 2017, "Neuropathic pain", Nat Rev Dis Primers, Vol. 3, p. 17002.
Constandil et al., 2011, "Involvement of spinal cord BDNF in the generation and maintenance of chronic neuropathic pain in rats", Brain Res Bull, Vol. 86, No. 5-6, pp. 454-459.
Geng et al., 2010, "Contribution of the spinal cord BDNF to the development of neuropathic pain by activation of the NR2B-containing NMDA receptors in rats with spinal nerve ligation", Exp Neurol, Vol. 222, No. 2, pp. 256-266.
Goncalves et al., 2019a, "Discovery and lead optimisation of a potent, selective and orally bioavailable RARβ agonist for the potential treatment of nerve injury", Bio-Org & Med. Chem. Lett., vol. 29, no. 8, pp. 995-1000 (https://doi.org/10.1016/j.bmcl.2019.02.011) (available online 11 February 2019).
Goncalves et al., 2019b, "RARβ Agonist Drug (C286) Demonstrates Efficacy in a Pre-clinical Neuropathic Pain Model Restoring Multiple Pathways via DNA Repair Mechanisms", iScience, vol. 20, pp. 554-566 (https://doi.org/10.1016/j.isci.2019.09.020) (available online 17 September 2019).
Hargreaves et al., 1998, "A new and sensitive method for measuring thermal nociception in cutaneous hyperalgesia", Pain, Vol. 32, No. 1, p. 77-88.
Huang et al., 2016, "A Comparison of Surgical Invasions for Spinal Nerve Ligation with or without Paraspinal Muscle Removal in a Rat Neuropathic Pain Model", Biomed Res Int, p. 6741295
Jensen et al., 2014, "Allodynia and hyperalgesia in neuropathic pain: clinical manifestations and mechanisms", Lancet Neurol, Vol. 13, No. 9, pp. 924-935.
Kim et al., 1992, "An experimental model for peripheral neuropathy produced by segmental spinal nerve ligation in the rat", Pain, Vol. 50, No. 3, pp. 355-363.
Meacham et al., 2017, "Neuropathic Pain: Central vs. Peripheral Mechanisms", Curr Pain Headache Rep, Vol. 21, No. 6, p. 28.
Raghavendra et al., 2003, "Inhibition of microglial activation attenuates the development but not existing hypersensitivity in a rat model of neuropathy", J. Pharmacol. Exp. Ther., Vol. 306, pp. 624-630.
Schafers et al., 2003A, "Tumor necrosis factor-alpha induces mechanical allodynia after spinal nerve ligation by activation of p38 MAPK in primary sensory neurons", J Neurosci, Vol. 23, No. 7, pp. 2517-2521.
Schafers et al., 2003B, "Spinal nerve ligation induces transient upregulation of tumor necrosis factor receptors 1 and 2 in injured and adjacent uninjured dorsal root ganglia in the rat", Neurosci Lett, Vol. 347, No. 3, pp. 179-182.
Seltzer et al., 1990, "A novel behavioral model of neuropathic pain disorders produced in rats by partial sciatic nerve injury", Pain, Vol. 43, No. 2, pp. 205-218.

## Claims

1. A compound of the following formula, or a pharmaceutically acceptable salt, hydrate, or solvate thereof: 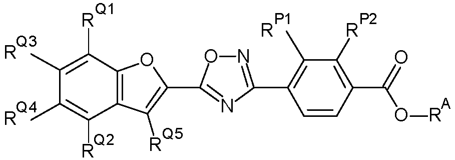 wherein:
-R^{Q1} is independently -R^{Q1A}, -R^{Q1B}, or -R^{Q1C};
-R^{Q1A} is saturated linear or branched C₁₋₄alkyl;
-R^{Q1B} is independently -F, -Cl, -Br, or -I;
-R^{Q1C} is -CF₃;
-R^{Q2} is independently -R^{Q2A}, -R^{Q2B}, or -R^{Q2C};
-R^{Q2A} is saturated linear or branched C₁₋₄alkyl;
-R^{Q2B} is independently -F, -Cl, -Br, or -I;
-R^{Q2C} is -CF₃;
-R^{Q3} is independently -H, -R^{Q3A}, -R^{Q3B}, or -R^{Q3C};
-R^{Q3A} is saturated linear or branched C₁₋₄alkyl;
-R^{Q3B} is independently -F, -Cl, -Br, or -I;
-R^{Q3C} is -CF₃;
-R^{Q4} is independently -H, -R^{Q4A}, -R^{Q4B}, or -R^{Q4C};
-R^{Q4A} is saturated linear or branched C₁₋₄alkyl;
-R^{Q4B} is independently -F, -Cl, -Br, or -I;
-R^{Q4C} is -CF₃;
-R^{Q5} is independently -H, -R^{Q5A}, -R^{Q5B}, or -R^{Q5C};
-R^{Q5A} is saturated linear or branched C₁₋₄alkyl;
-R^{Q5B} is independently -F, -Cl, -Br, or -I;
-R^{Q5C} is -CF₃;
-R^{P1} is independently -H or -R^{P1X};
-R^{P1X} is independently -R^{P1A}, -R^{P1B}, -R^{P1C}, or -R^{P1D};
-R^{P1A} is saturated linear or branched C₁₋₄alkyl;
-R^{P1B} is independently -F, -Cl, -Br, or -I;
-R^{P1C} is -CF₃;
-R^{P1D} is independently -NH₂, -NHR^{P1DD}, or -NR^{P1DD}₂;
-R^{P1DD} is saturated linear or branched C₁₋₄alkyl;
-R^{P2} is independently -H or -R^{P2X};
-R^{P2X} is independently -R^{P2A}, -R^{P2B}, -R^{P2C}, or -R^{P2D};
-R^{P2A} is saturated linear or branched C₁₋₄alkyl;
-R^{P2B} is independently -F, -Cl, -Br, or -I;
-R^{P2C} is -CF₃;
-R^{P2D} is independently -NH₂, -NHR^{P2DD}, or -NR^{P2DD}₂;
-R^{P2DD} is saturated linear or branched C₁₋₄alkyl;
-R^{A} is independently -H or -R^{AA}; and
-R^{AA} is independently saturated linear or branched C₁₋₄alkyl, phenyl, or benzyl;
or a pharmaceutical composition comprising such a compound and a pharmaceutically acceptable carrier, diluent, or excipient;
for use in a method of treatment or prevention of neuropathic pain.

2. A compound or pharmaceutical composition for use according to claim 1,
wherein -R^{Q1} is -R^{Q1A}.

3. A compound or pharmaceutical composition for use according to claim 1 or 2,
wherein -R^{Q2} is -R^{Q2A}.

4. A compound or pharmaceutical composition for use according to any one of claims 1 to 3,
wherein -R^{Q3} is -H.

5. A compound or pharmaceutical composition for use according to any one of claims 1 to 4,
wherein -R^{Q4} is -H.

6. A compound or pharmaceutical composition for use according to any one of claims 1 to 5,
wherein -R^{Q5} is -H.

7. A compound or pharmaceutical composition for use according to any one of claims 1 to 6,
wherein -R^{Q1A} is -Me.

8. A compound or pharmaceutical composition for use according to any one of claims 1 to 7,
wherein -R^{Q2A} is -Me.

9. A compound or pharmaceutical composition for use according to any one of claims 1 to 8,
wherein -R^{P1} is -H.

10. A compound or pharmaceutical composition for use according to any one of claims 1 to 9,
wherein -R^{P2} is -H.

11. A compound or pharmaceutical composition for use according to any one of claims 1 to 10,
wherein -R^{A} is -H.

12. A compound or pharmaceutical composition for use according to any one of claims 1 to 11,
wherein:
-R^{Q3} is -H;
-R^{Q4} is -H;
-R^{Q5} is -H;
-R^{P1} is -H;
-R^{P2} is -H; and
-R^{A} is -H.

13. A compound or pharmaceutical composition for use according to claim 1, wherein the compound is a compound of one of the following formulae, or a pharmaceutically acceptable salt, hydrate, or solvate thereof: 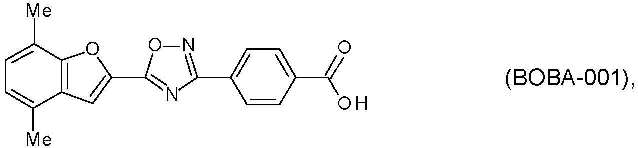 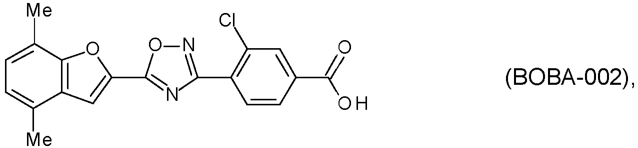 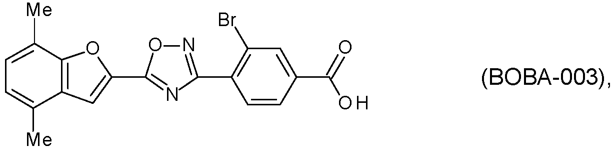 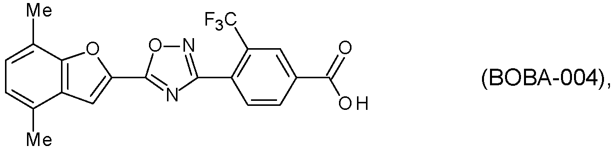 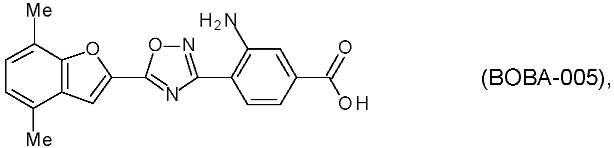 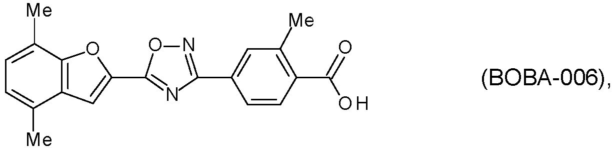 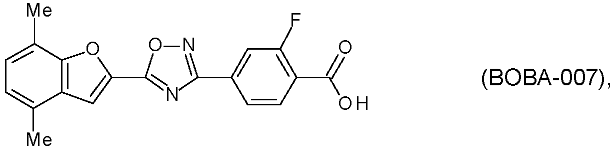 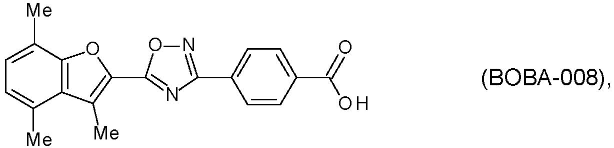 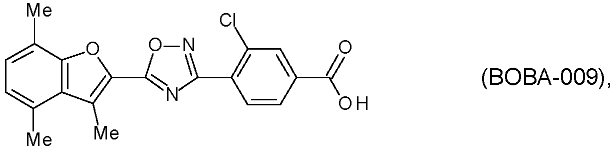 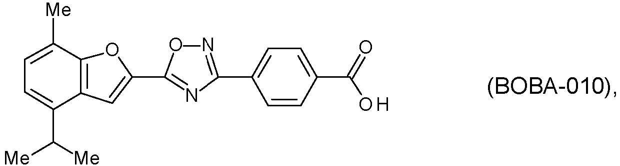 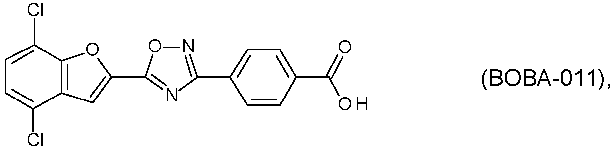 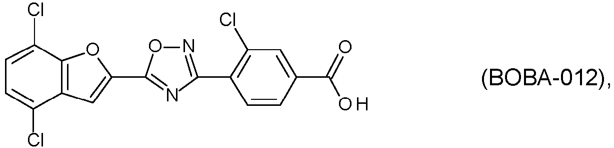 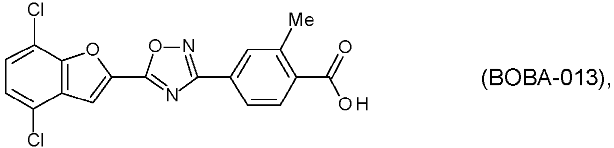 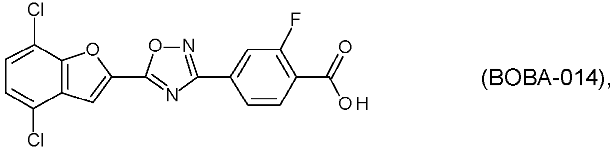 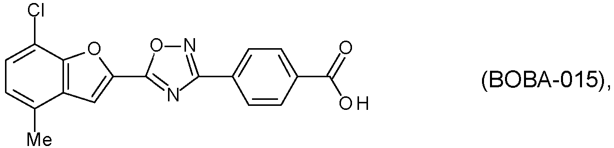 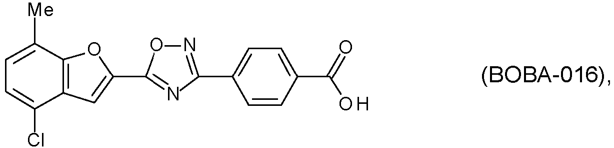 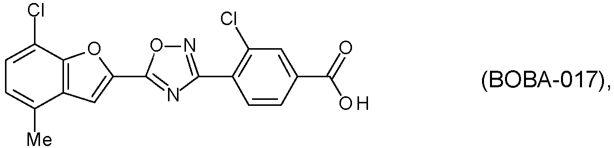 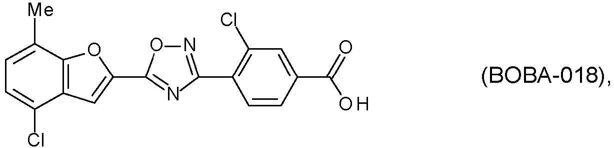 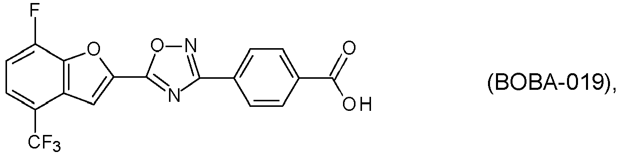 and

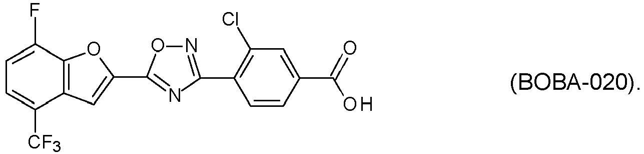

14. A compound or pharmaceutical composition for use according to claim 1,
wherein the compound is a compound of the following formula, or a pharmaceutically acceptable salt, hydrate, or solvate thereof:

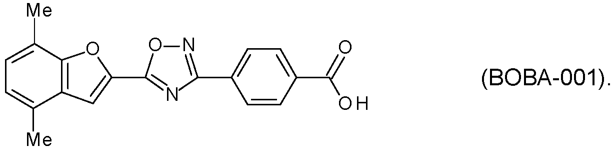

15. A compound or pharmaceutical composition for use according to any one of claims 1 to 14,
wherein the neuropathic pain is:
peripheral neuropathic pain;
central neuropathic pain;
chronic neuropathic pain;
refractory neuropathic pain;
neuropathic pain associated with a metabolic dysfunction, including, for example, diabetes mellitus and pre-diabetes;
neuropathic pain associated with diabetes mellitus;
neuropathic pain associated with pre-diabetes;
neuropathic pain associated with painful polyneuropathy;
neuropathic pain associated with painful diabetic neuropathy, including, for example, diabetic peripheral neuropathy;
neuropathic pain associated with painful diabetic polyneuropathy;
neuropathic pain associated with post-herpetic neuralgia;
neuropathic pain associated with trigeminal neuralgia;
neuropathic pain associated with occipital neuralgia;
neuropathic pain associated with painful radiculopathy, including, for example, lumbar and cervical painful radiculopathy;
neuropathic pain associated with an infectious disease, including, for example, herpes zoster (shingles), HIV infection, Lyme disease, diphtheria, and leprosy;
neuropathic pain associated with a liver or kidney disorder, including, for example, a chronic liver or kidney disorder, including, for example, liver disease, liver failure, kidney disease, and kidney failure;
neuropathic pain associated with an immune or inflammatory disorder, including, for example, Guillain-Barré syndrome, rheumatoid arthritis, lupus, Sjörgren's syndrome, and coeliac disease;
neuropathic pain associated with an inherited neuropathy or channelopathy, including, for example, inherited erythromelalgia, paroxysmal extreme pain disorder, and Charcot-Marie-Tooth disease (CMT);
neuropathic pain associated with small fibre sensory neuropathy;
neuropathic pain associated with a thyroid hormone disorder, including, for example, hypothyroidism;
neuropathic pain associated with stroke;
neuropathic pain associated with cancer, including, for example, lymphoma and multiple myeloma;
neuropathic pain associated with chemotherapy, for example, cancer chemotherapy;
neuropathic pain associated with peripheral nerve injury pain;
neuropathic pain associated with nerve damage following traumatic injury;
neuropathic pain associated with post-traumatic neuropathy;
neuropathic pain associated with spinal cord injury, including, for example, spinal cord injury caused by trauma, for example, a road traffic accident;
neuropathic pain associated with traumatic peripheral nerve injury;
neuropathic pain associated with post-surgery neuropathy (e.g., post-surgery neuropathic pain);
neuropathic pain following surgery, including, for example, neuropathic pain following nerve surgery, including, for example, spinal cord surgery;
neuropathic pain associated with fibromyalgia;
neuropathic pain associated with lower back pain;
neuropathic pain associated with carpal tunnel syndrome;
neuropathic pain associated with causalgia;
neuropathic pain associated with reflex sympathetic dystrophy (RSD);
neuropathic pain associated with Complex Regional Pain Syndrome (CRPS), including, for example, Type 1 and Type 2;
neuropathic pain associated with amputation;
neuropathic pain associated with a neurodegenerative disease, for example, Parkinson's disease;
neuropathic pain associated with stroke, including, for example, central post-stroke pain;
neuropathic pain associated with syringomyelia;
neuropathic pain associated with a demyelinating disease, including, for example, multiple sclerosis, transverse myelitis, and neuromyelitis optica; or
idiopathic neuropathic pain.

16. A compound or pharmaceutical composition for use according to any one of claims 1 to 13, wherein the neuropathic pain is:
peripheral neuropathic pain.

## Patentansprüche

1. Verbindung der folgenden Formel oder ein pharmazeutisch annehmbares Salz, Hydrat oder Solvat davon: 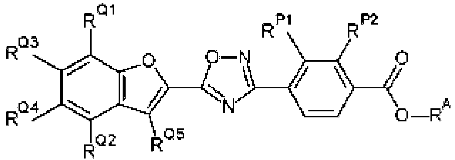 worin:
-R^{Q1} jeweils unabhängig -R^{Q1A}, -R^{Q1B} oder -R^{Q1C} ist;
-R^{Q1A} gesättigtes, unverzweigtes oder verzweigtes C₁₋₄-Alkyl ist;
-R^{Q1B} jeweils unabhängig -F, -Cl, -Br oder -I ist;
-R^{Q1C} -CF₃ ist;
-R^{Q2} jeweils unabhängig -R^{Q2A}, R^{Q2B} oder R^{Q2C} ist;
-R^{Q2A} gesättigtes, unverzweigtes oder verzweigtes C₁₋₄-Alkyl ist;
-R^{Q2B} jeweils unabhängig -F, -Cl, -Br oder -I ist;
-R^{Q2C} -CF₃ ist;
-R^{Q3} jeweils unabhängig -H, -R^{Q3A}, R^{Q3B} oder R^{Q3C} ist;
-R^{Q3A} gesättigtes, unverzweigtes oder verzweigtes C₁₋₄-Alkyl ist;
-R^{Q3B} jeweils unabhängig -F, -Cl, -Br oder -I ist;
-R^{Q3C} -CF₃ ist;
-R^{Q4} jeweils unabhängig -H, -R^{Q4A}, R^{Q4B} oder R^{Q4C} ist;
-R^{Q4A} gesättigtes, unverzweigtes oder verzweigtes C₁₋₄-Alkyl ist;
-R^{Q4B} jeweils unabhängig -F, -Cl, -Br oder -I ist;
-R^{Q4C} -CF₃ ist;
-R^{Q5} jeweils unabhängig -H, -R^{Q5A}, R^{Q5B} oder R^{Q5C} ist;
-R^{Q5A} gesättigtes, unverzweigtes oder verzweigtes C₁₋₄-Alkyl ist;
-R^{Q5B} jeweils unabhängig -F, -Cl, -Br oder -I ist;
-R^{Q5C} -CF₃ ist;
-R^{P1} jeweils unabhängig -H oder -R^{P1X} ist;
-R^{P1X} jeweils unabhängig -R^{P1A}, -R^{P1B}, -R^{P1C} oder -R^{P1D} ist;
-R^{P1A} gesättigtes, unverzweigtes oder verzweigtes C₁₋₄-Alkyl ist;
-R^{P1B} jeweils unabhängig -F, -Cl, -Br oder -I ist;
-R^{P1C} -CF₃ ist;
-R^{P1D} jeweils unabhängig -NH₂, -NHR^{P1DD} oder -NR^{P1DD}₂ ist;
-R^{P1DD} gesättigtes, unverzweigtes oder verzweigtes C₁₋₄-Alkyl ist;
-R^{P2} jeweils unabhängig -H oder -R^{P2X} ist;
-R^{P2X} jeweils unabhängig -R^{P2A}, -R^{P2B}, -R^{P2C} oder -R^{P2D} ist;
-R^{P2A} gesättigtes, unverzweigtes oder verzweigtes C₁₋₄-Alkyl ist;
-R^{P2B} jeweils unabhängig -F, -Cl, -Br oder -I ist;
-R^{P2C} -CF₃ ist;
-R^{P2D} jeweils unabhängig -NH₂, -NHR^{P2DD} oder -NR^{P2DD}₂ ist;
-R^{P2DD} gesättigtes, unverzweigtes oder verzweigtes C₁₋₄-Alkyl ist;
-R^{A} jeweils unabhängig -H oder -R^{AA} ist; und
-R^{AA} jeweils unabhängig gesättigtes, unverzweigtes oder verzweigtes C₁₋₄-Alkyl, Phenyl oder Benzyl ist;
oder eine pharmazeutische Zusammensetzung, die eine derartige Verbindung und einen pharmazeutisch annehmbaren Träger, Verdünner oder Hilfsstoff umfasst;
zur Verwendung in einem Verfahren zur Behandlung oder Prävention von neuropathischen Schmerzen.

2. Verbindung oder pharmazeutische Zusammensetzung zur Verwendung nach Anspruch 1,
worin -R^{Q1} -R^{Q1A} ist.

3. Verbindung oder pharmazeutische Zusammensetzung zur Verwendung nach Anspruch 1 oder 2,
worin -R^{Q2} -R^{Q2A} ist.

4. Verbindung oder pharmazeutische Zusammensetzung zur Verwendung nach einem der Ansprüche 1 bis 3,
worin -R^{Q3} -H ist.

5. Verbindung oder pharmazeutische Zusammensetzung zur Verwendung nach einem der Ansprüche 1 bis 4,
worin -R^{Q4} -H ist.

6. Verbindung oder pharmazeutische Zusammensetzung zur Verwendung nach einem der Ansprüche 1 bis 5,
worin -R^{Q5} -H ist.

7. Verbindung oder pharmazeutische Zusammensetzung zur Verwendung nach einem der Ansprüche 1 bis 6,
worin -R^{Q1A} -Me ist.

8. Verbindung oder pharmazeutische Zusammensetzung zur Verwendung nach einem der Ansprüche 1 bis 7,
worin -R^{Q2A} -Me ist.

9. Verbindung oder pharmazeutische Zusammensetzung zur Verwendung nach einem der Ansprüche 1 bis 8,
worin -R^{P1} -H ist.

10. Verbindung oder pharmazeutische Zusammensetzung zur Verwendung nach einem der Ansprüche 1 bis 9,
worin -R^{P2} -H ist.

11. Verbindung oder pharmazeutische Zusammensetzung zur Verwendung nach einem der Ansprüche 1 bis 10,
worin -R^{A} -H ist.

12. Verbindung oder pharmazeutische Zusammensetzung zur Verwendung nach einem der Ansprüche 1 bis 11,
worin:
-R^{Q3} -H ist;
-R^{Q4} -H ist;
-R^{Q5} -H ist;
-R^{P1} -H ist;
-R^{P2} -H ist; und
-R^{A} -H ist.

13. Verbindung oder pharmazeutische Zusammensetzung zur Verwendung nach Anspruch 1, wobei die Verbindung eine Verbindung einer der folgenden Formeln oder ein pharmazeutisch annehmbares Salz, Hydrat oder Solvat davon ist: 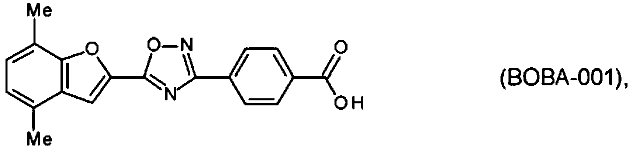 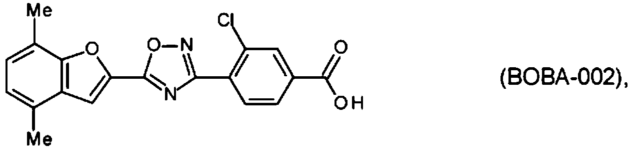 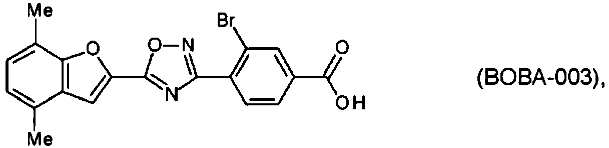 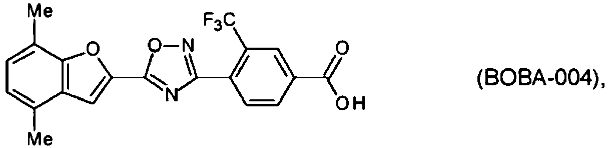 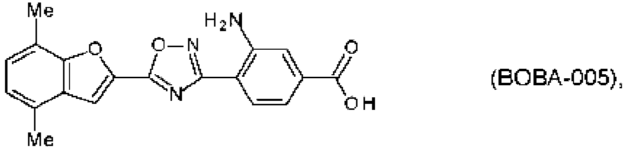 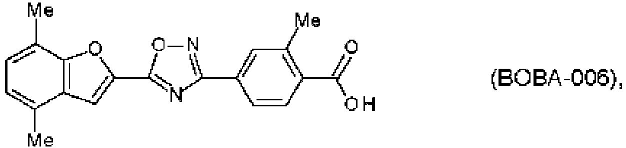 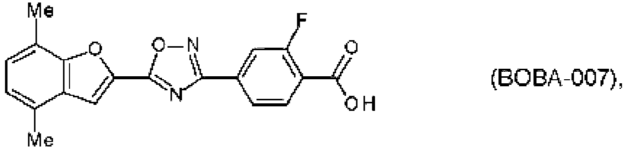 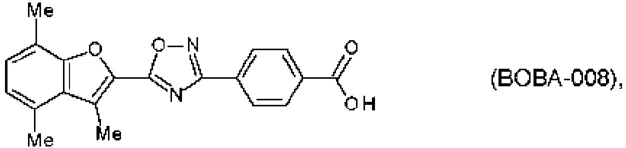 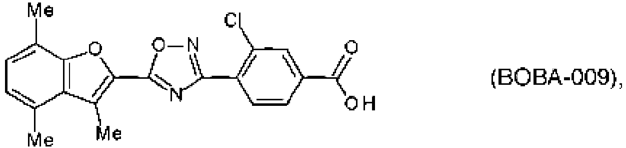 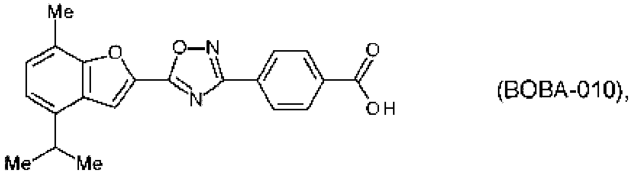 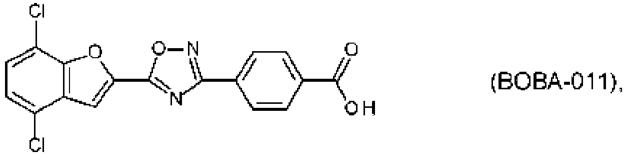 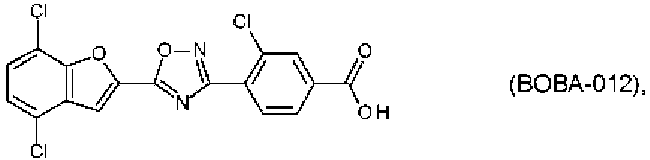 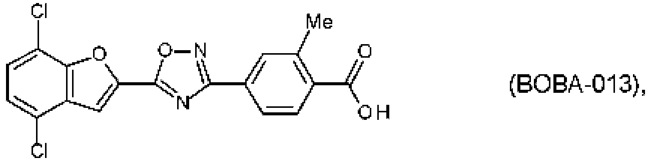 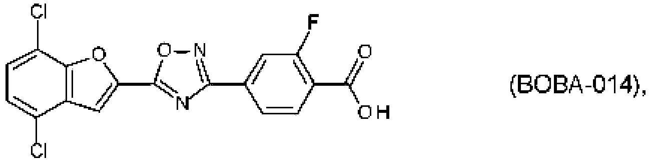 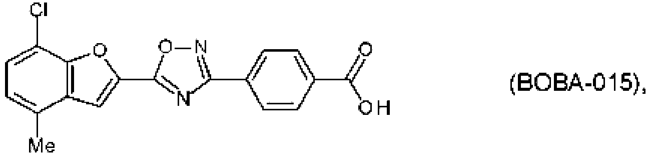 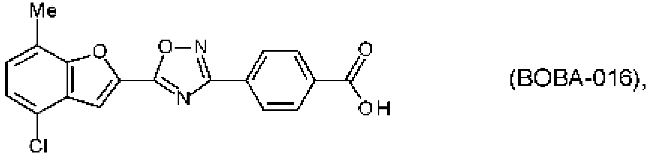 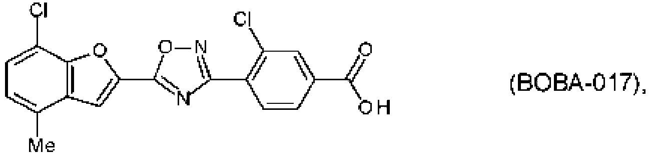 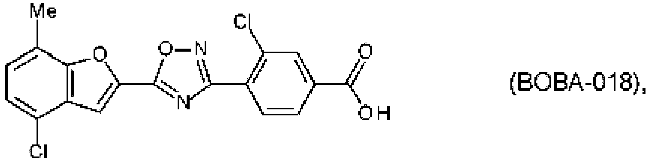 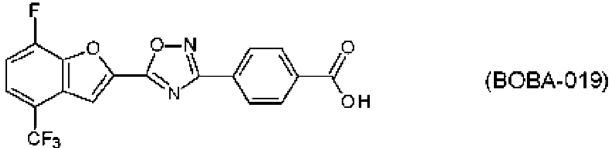 und

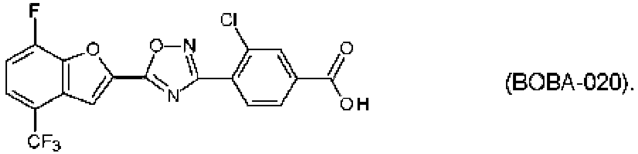

14. Verbindung oder pharmazeutische Zusammensetzung zur Verwendung nach Anspruch 1, wobei die Verbindung eine Verbindung der folgenden Formel oder ein pharmazeutisch annehmbares Salz, Hydrat oder Solvat davon ist:

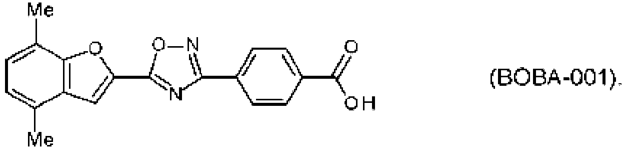

15. Verbindung oder pharmazeutische Zusammensetzung zur Verwendung nach einem der Ansprüche 1 bis 14,
wobei die neuropathischen Schmerzen die folgenden sind:
periphere neuropathische Schmerzen;
zentrale neuropathische Schmerzen;
chronische neuropathische Schmerzen;
refraktäre neuropathische Schmerzen;
neuropathische Schmerzen im Zusammenhang mit einer Stoffwechselstörung, einschließlich von z. B. Diabetes mellitus und Prädiabetes;
neuropathische Schmerzen im Zusammenhang mit Diabetes mellitus;
neuropathische Schmerzen im Zusammenhang mit Prädiabetes;
neuropathische Schmerzen im Zusammenhang mit schmerzhafter Polyneuropathie;
neuropathische Schmerzen im Zusammenhang mit schmerzhafter diabetischer Neuropathie, einschließlich von z. B. diabetischer peripherer Neuropathie;
neuropathische Schmerzen im Zusammenhang mit schmerzhafter diabetischer Polyneuropathie;
neuropathische Schmerzen im Zusammenhang mit postherpetischer Neuralgie;
neuropathische Schmerzen im Zusammenhang mit Trigeminusneuralgie;
neuropathische Schmerzen im Zusammenhang mit Okzipitalneuralgie;
neuropathische Schmerzen im Zusammenhang mit schmerzhafter Radikulopathie, einschließlich von z. B. lumbaler und zervikaler schmerzhafter Radikulopathie;
neuropathische Schmerzen im Zusammenhang mit einer Infektionskrankheit, einschließlich von z. B. Herpes zoster (Gürtelrose), HIV-Infektion, Lyme-Borreliose, Diphtherie und Lepra;
neuropathische Schmerzen im Zusammenhang mit einer Leber- oder Nierenerkrankung, einschließlich von z. B. einer chronischen Leber- oder Nierenerkrankung, wie z. B. Lebererkrankung, Leberversagen, Nierenerkrankungen und Nierenversagen;
neuropathische Schmerzen im Zusammenhang mit einer Immun- oder Entzündungserkrankung, einschließlich von z. B. dem Guillain-Barré-Syndrom, rheumatoider Arthritis, Lupus, dem Sjögren-Syndrom und Zöliakie;
neuropathische Schmerzen im Zusammenhang mit einer erblichen Neuropathie oder Kanalopathie, einschließlich von z. B. vererbter Erythromelalgie, paroxysmaler extremer Schmerzstörung und der Charcot-Marie-Tooth-Krankheit (CMT);
neuropathische Schmerzen im Zusammenhang mit einer sensorischen Neuropathie der kleinen Nervenfasern;
neuropathische Schmerzen im Zusammenhang mit einer Schilddrüsenhormonstörung, einschließlich von z. B. Hypothyreose;
neuropathische Schmerzen im Zusammenhang mit einem Schlaganfall;
neuropathische Schmerzen im Zusammenhang mit Krebs, einschließlich von z. B. Lymphomen und multiplem Myelom;
neuropathische Schmerzen im Zusammenhang mit einer Chemotherapie, z. B. einer Krebs-Chemotherapie;
neuropathische Schmerzen im Zusammenhang mit einer Verletzung peripherer Nerven;
neuropathische Schmerzen im Zusammenhang mit einer Nervenschädigung nach einer traumatischen Verletzung;
neuropathische Schmerzen im Zusammenhang mit einer posttraumatischen Neuropathie;
neuropathische Schmerzen im Zusammenhang mit einer Rückenmarksverletzung, einschließlich von z. B. einer durch ein Trauma, wie z. B. einen Verkehrsunfall, verursachten Rückenmarksverletzung;
neuropathische Schmerzen im Zusammenhang mit einer traumatischen peripheren Nervenverletzung;
neuropathische Schmerzen im Zusammenhang mit einer postoperativen Neuropathie (z. B. postoperative neuropathische Schmerzen);
neuropathische Schmerzen nach einer Operation, einschließlich von z. B. neuropathischen Schmerzen nach einer Nervenoperation, einschließlich von z. B. einer Rückenmarksoperation;
neuropathische Schmerzen im Zusammenhang mit Fibromyalgie;
neuropathische Schmerzen im Zusammenhang mit Schmerzen im unteren Rückenbereich;
neuropathische Schmerzen im Zusammenhang mit dem Karpaltunnelsyndrom;
neuropathische Schmerzen im Zusammenhang mit Kausalgie;
neuropathische Schmerzen im Zusammenhang mit reflexsympathischer Dystrophie (RSD);
neuropathische Schmerzen im Zusammenhang mit komplexem regionalen Schmerzsyndrom (CRPS), einschließlich von z. B. Typ 1 und Typ 2;
neuropathische Schmerzen im Zusammenhang mit einer Amputation;
neuropathische Schmerzen im Zusammenhang mit einer neurodegenerativen Erkrankung, z. B. der Parkinson-Krankheit;
neuropathische Schmerzen im Zusammenhang mit einem Schlaganfall, einschließlich von z. B. zentralen post-schlaganfallbedingten Schmerzen;
neuropathische Schmerzen im Zusammenhang mit Syringomyelie;
neuropathische Schmerzen im Zusammenhang mit einer demyelinisierenden Erkrankung, einschließlich von z. B. multipler Sklerose, transversaler Myelitis und Neuromyelitis optica; oder
idiopathische neuropathische Schmerzen.

16. Verbindung oder pharmazeutische Zusammensetzung zur Verwendung nach einem der Ansprüche 1 bis 13, wobei die neuropathischen Schmerzen die folgenden sind:
periphere neuropathische Schmerzen.

## Revendications

1. Composé de la formule suivante, ou un sel, un hydrate ou un solvate pharmaceutiquement acceptable de celui-ci :
dans lequel : 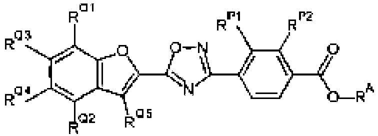
-R^{Q1} est indépendamment -R^{Q1A}, -R^{Q1B} ou -R^{Q1C} ;
-R^{Q1A} est un alkyle en C₁₋₄ linéaire ou ramifié saturé ;
-R^{Q1B} est indépendamment -F, -Cl, -Br ou -I ;
-R^{Q1C} est -CF₃ ;
-R^{Q2} est indépendamment -R^{Q2A}, -R^{Q2B} ou -R^{Q2C} ;
-R^{Q2A} est un alkyle en C₁₋₄ linéaire ou ramifié saturé ;
-R^{Q2B} est indépendamment -F, -Cl, -Br ou -I ;
-R^{Q2C} est -CF₃ ;
-R^{Q3} est indépendamment -H, -RQ3A, -R^{Q3B} ou -R^{Q3C};
-R^{Q3A} est un alkyle en C₁₋₄ linéaire ou ramifié saturé ;
-R^{Q3B} est indépendamment -F, -Cl, -Br ou -I ;
-R^{Q3C} est -CF₃ ;
-R^{Q4} est indépendamment -H, -R_{Q4A}, -R_{Q4B} ou -R_{Q4C} ;
-R^{Q4A} est un alkyle en C₁₋₄ linéaire ou ramifié saturé ;
-R^{Q4B} est indépendamment -F, -Cl, -Br ou -I ;
-R^{Q4C} est -CF₃ ;
-R^{Q5} est indépendamment -H, -R^{Q5A}, -R^{Q5B} ou -R^{Q5C} ;
-R^{Q5A} est un alkyle en C₁₋₄ linéaire ou ramifié saturé ;
-R^{Q5B} est indépendamment -F, -Cl, -Br ou -I ;
-R^{Q5C} est -CF₃ ;
-R^{P1} est indépendamment -H ou -R^{P1X} ;
-R^{P1X} est indépendamment -R^{P1A}, -R^{P1B}, -R^{P1C} ou -R^{P1D} ;
-R^{P1A} est un alkyle en C₁₋₄ linéaire ou ramifié saturé;
-R^{P1B} est indépendamment -F, -Cl, -Br ou -I ;
-R^{P1C} est -CF₃ ;
-R^{P1D} est indépendamment -NH₂, -NHR^{P1DD} ou -NR^{P1DD}₂ ;
-R^{P1DD} est un alkyle en C₁₋₄ linéaire ou ramifié saturé ;
-R^{P2} est indépendamment -H ou -R^{P2X} ;
-R^{P2X} est indépendamment -R^{P2A}, -R^{P2B}, -R^{P2C} ou -R^{P2D} ;
-R^{P2A} est un alkyle en C₁₋₄ linéaire ou ramifié saturé ;
-R^{P2B} est indépendamment -F, -Cl, -Br ou -I ;
-R^{P2C} est -CF₃ ;
-R^{P2D} est indépendamment -NH₂, -NHR^{P2DD} ou -NR^{P2DD}₂ ;
-R^{P2DD} est un alkyle en C₁₋₄ linéaire ou ramifié saturé ;
-R^{A} est indépendamment -H ou -R^{AA} ; et
-R^{AA} est indépendamment un alkyle en C₁₋₄ linéaire ou ramifié saturé, un phényle ou un benzyle ;
ou une composition pharmaceutique comprenant un tel composé et un support, un diluant ou un excipient pharmaceutiquement acceptable ; pour utilisation dans un procédé de traitement ou de prévention de la douleur neuropathique.

2. Composé ou composition pharmaceutique pour utilisation selon la revendication 1, -R^{Q1} étant -R^{Q1A}.

3. Composé ou composition pharmaceutique pour utilisation selon la revendication 1 ou 2, -R^{Q2} étant -R^{Q2A}

4. Composé ou composition pharmaceutique pour utilisation selon l'une quelconque des revendications 1 à 3, -R^{Q3} étant -H.

5. Composé ou composition pharmaceutique pour utilisation selon l'une quelconque des revendications 1 à 4, -R^{Q4} étant -H.

6. Composé ou composition pharmaceutique pour utilisation selon l'une quelconque des revendications 1 à 5, -R^{Q5} étant -H.

7. Composé ou composition pharmaceutique pour utilisation selon l'une quelconque des revendications 1 à 6, -R^{Q1A} étant -Me

8. Composé ou composition pharmaceutique pour utilisation selon l'une quelconque des revendications 1 à 7, -R^{Q2A} étant -Me

9. Composé ou composition pharmaceutique pour utilisation selon l'une quelconque des revendications 1 à 8, -R^{P1} étant -H.

10. Composé ou composition pharmaceutique pour utilisation selon l'une quelconque des revendications 1 à 9, -R^{P2} étant -H.

11. Composé ou composition pharmaceutique pour utilisation selon l'une quelconque des revendications 1 à 10, -R^{A} étant -H.

12. Composé ou composition pharmaceutique pour utilisation selon l'une quelconque des revendications 1 à 11,
-R^{Q3} étant -H ;
-R^{Q4} étant -H ;
-R^{Q5} étant -H ;
-R^{P1} étant -H ;
-R_{P2} étant -H ; et
-R^{A} étant -H.

13. Composé ou composition pharmaceutique pour utilisation selon la revendication 1, le composé étant un composé de l'une des formules suivantes, ou un sel, un hydrate ou un solvate pharmaceutiquement acceptable de celui-ci : 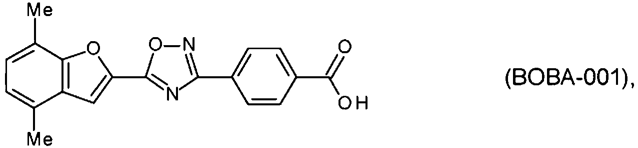 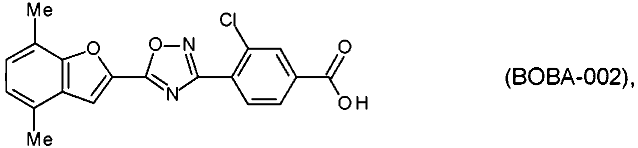 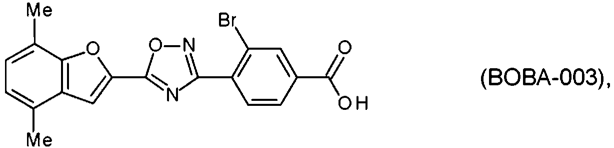 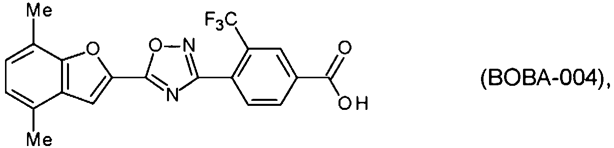 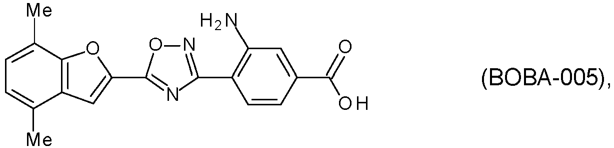 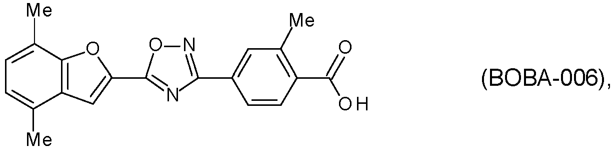 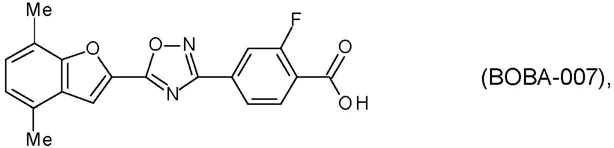 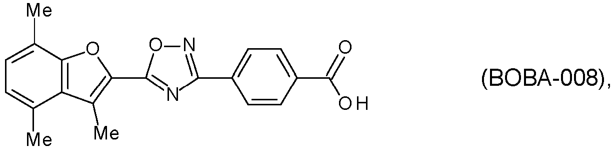 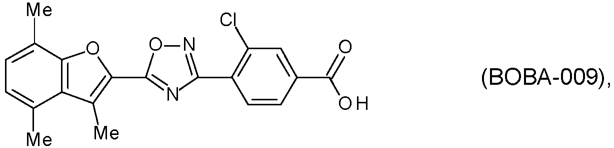 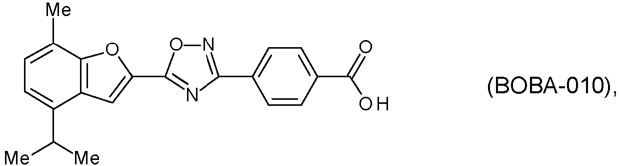 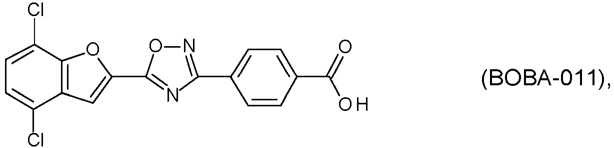 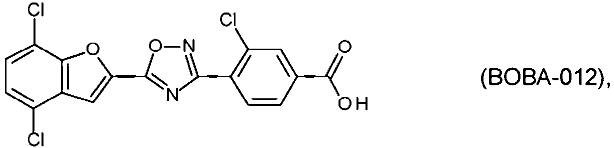 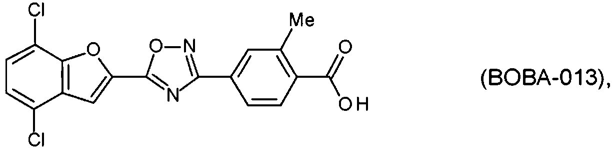 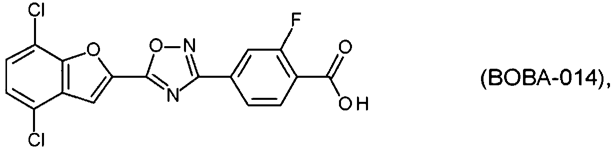 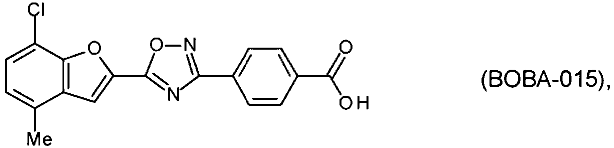 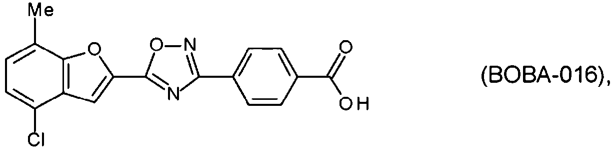 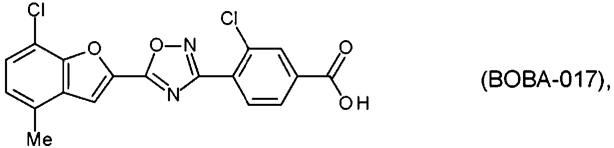 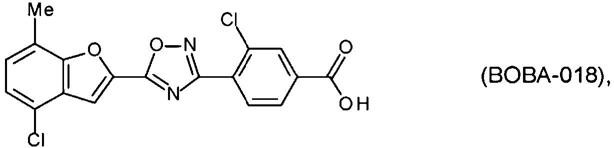 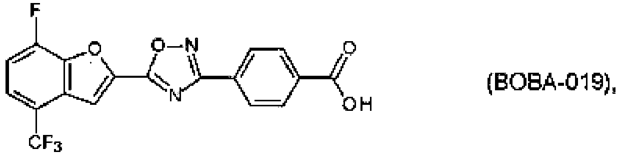 et

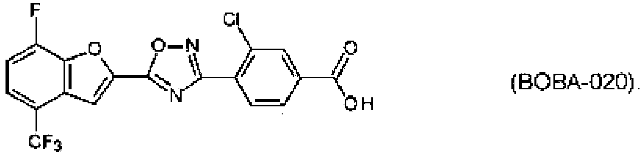

14. Composé ou composition pharmaceutique pour utilisation selon la revendication 1, le composé étant un composé de la formule suivante, ou un sel, un hydrate ou un solvate pharmaceutiquement acceptable de celui-ci :

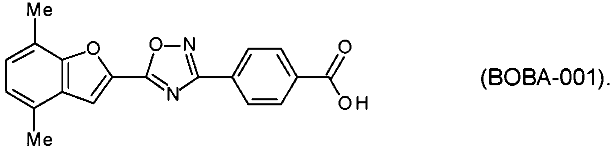

15. Composé ou composition pharmaceutique pour utilisation selon l'une quelconque des revendications 1 à 14, dans lequel la douleur neuropathique est ;
une douleur neuropathique périphérique ;
une douleur neuropathique centrale ;
une douleur neuropathique chronique ;
une douleur neuropathique réfractaire ;
une douleur neuropathique associée à un dysfonctionnement métabolique, y compris, par exemple, le diabète sucré et le prédiabète ;
une douleur neuropathique associée au diabète sucré ;
une douleur neuropathique associée au prédiabète ;
une douleur neuropathique associée à une polyneuropathie douloureuse ;
une douleur neuropathique associée à une neuropathie diabétique douloureuse, y compris, par exemple, une neuropathie périphérique diabétique ;
une douleur neuropathique associée à une polyneuropathie diabétique douloureuse ;
une douleur neuropathique associée à une névralgie post-herpétique ;
une douleur neuropathique associée à une névralgie du trijumeau ;
une douleur neuropathique associée à une névralgie occipitale ;
une douleur neuropathique associée à une radiculopathie douloureuse, y compris, par exemple, une radiculopathie douloureuse lombaire et cervicale ;
une douleur neuropathique associée à une maladie infectieuse, y compris, par exemple, l'herpès zoster (zona), l'infection par le VIH, la maladie de Lyme, la diphtérie et la lèpre ;
une douleur neuropathique associée à une maladie du foie ou des reins, y compris, par exemple, une maladie chronique du foie ou des reins, y compris, par exemple, une maladie du foie, une insuffisance hépatique, une maladie rénale et une insuffisance rénale ;
une douleur neuropathique associée à un trouble immunitaire ou inflammatoire, y compris, par exemple, le syndrome de Guillain-Barré, la polyarthrite rhumatoïde, le lupus, le syndrome de Sjörgren et la maladie cœliaque ;
une douleur neuropathique associée à une neuropathie ou à une canalopathie héréditaire, y compris, par exemple, une érythromélalgie héréditaire, un trouble douloureux extrême paroxystique et une maladie de Charcot-Marie-Tooth (CMT) ;
une douleur neuropathique associée à une neuropathie sensorielle des petites fibres ;
une douleur neuropathique associée à un trouble hormonal thyroïdien, y compris, par exemple, une hypothyroïdie ;
une douleur neuropathique associée à un accident vasculaire cérébral ;
une douleur neuropathique associée au cancer, y compris, par exemple, un lymphome et un myélome multiple ;
une douleur neuropathique associée à une chimiothérapie, par exemple, une chimiothérapie anticancéreuse ;
une douleur neuropathique associée à une douleur liée à une lésion nerveuse périphérique ;
une douleur neuropathique associée à des lésions nerveuses suite à une lésion traumatique ;
une douleur neuropathique associée à une neuropathie post-traumatique ;
une douleur neuropathique associée à une lésion de la moelle épinière, y compris, par exemple, une lésion de la moelle épinière causée par un traumatisme, par exemple, un accident de la route ;
une douleur neuropathique associée à une lésion traumatique du nerf périphérique ;
une douleur neuropathique associée à une neuropathie postopératoire (par exemple, une douleur neuropathique postopératoire) ;
une douleur neuropathique consécutive à une intervention chirurgicale, y compris, par exemple, une douleur neuropathique consécutive à une chirurgie nerveuse, y compris, par exemple, une chirurgie de la moelle épinière ;
une douleur neuropathique associée à la fibromyalgie ;
une douleur neuropathique associée à une douleur au bas du dos ;
une douleur neuropathique associée au syndrome du canal carpien ;
une douleur neuropathique associée à une causalgie ;
une douleur neuropathique associée à une dystrophie sympathique réflexe (DSR) ;
une douleur neuropathique associée au syndrome douloureux régional complexe (SDRC), y compris, par exemple, de type 1 et de type 2 ;
une douleur neuropathique associée à une amputation ;
une douleur neuropathique associée à une maladie neurodégénérative, par exemple la maladie de Parkinson ;
une douleur neuropathique associée à un accident vasculaire cérébral, y compris, par exemple, une douleur centrale post-accident vasculaire cérébral ;
une douleur neuropathique associée à une syringomyélie ;
une douleur neuropathique associée à une maladie démyélinisante, y compris, par exemple, la sclérose en plaques, la myélite transverse et la neuromyélite optique ; ou
une douleur neuropathique idiopathique.

16. Composé ou composition pharmaceutique pour utilisation selon l'une quelconque des revendications 1 à 13, la douleur neuropathique étant ;
une douleur neuropathique périphérique.
